(19) Europäisches Patentamt / European Patent Office / Office européen des brevets

(11) **EP 3 252 152 B1**

(12) **EUROPEAN PATENT SPECIFICATION**

(45) Date of publication and mention of the grant of the patent:
**22.07.2020  Bulletin 2020/30**

(51) Int Cl.:
*A61K 35/17* (2015.01)  *C12N 5/0783* (2010.01)
*A61P 35/00* (2006.01)  *A61P 35/02* (2006.01)
*A01N 1/00* (2006.01)

(21) Application number: **16743625.2**

(22) Date of filing: **15.01.2016**

(86) International application number:
**PCT/KR2016/000474**

(87) International publication number:
**WO 2016/122147 (04.08.2016 Gazette 2016/31)**

(54) **METHOD FOR MASS PRODUCING NATURAL KILLER CELL AND USE OF NATURAL KILLER CELL OBTAINED BY THE METHOD AS ANTI-CANCER AGENT**

VERFAHREN ZUR MASSENERZEUGUNG VON NATÜRLICHEN KILLERZELLEN UND VERWENDUNG NATÜRLICHER, DURCH DAS VERFAHREN HERGESTELLTER KILLERZELLEN ALS ANTIKREBSMITTEL

PROCÉDÉ PERMETTANT DE PRODUIRE EN MASSE UNE CELLULE TUEUSE NATURELLE ET UTILISATION DE LA CELLULE TUEUSES NATURELLE OBTENUE PAR LEDIT PROCÉDÉ EN TANT QU'AGENT ANTI-CANCÉREUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.01.2015  PCT/KR2015/000854**

(43) Date of publication of application:
**06.12.2017  Bulletin 2017/49**

(73) Proprietor: **Korea Research Institute of Bioscience and Biotechnology
Daejeon 34141 (KR)**

(72) Inventors:
• **CHOI, In Pyo
  Daejeon 34141 (KR)**
• **YOON, Suk Ran
  Daejeon 34141 (KR)**
• **LEE, Sooyun
  Daejeon 34141 (KR)**

(74) Representative: **Plougmann Vingtoft a/s
Strandvejen 70
2900 Hellerup (DK)**

(56) References cited:

EP-A2- 1 185 867   WO-A1-2013/094988
WO-A2-2010/013947   KR-A- 20100 045 704
KR-A- 20120 100 207   KR-A- 20140 051 263
US-A1- 2013 011 376   US-A1- 2014 023 626

• CHOI INPYO ET AL: "Donor-Derived Natural Killer Cells Infused after Human Leukocyte Antigen-Haploidentical Hematopoietic Cell Transplantation: A Dose-Escalation S", BIOLOGY OF BLOOD AND MARROW TRANSPLANTATION, vol. 20, no. 5, 2014, pages 696-704, XP028844979, ISSN: 1083-8791, DOI: 10.1016/J.BBMT.2014.01.031
• TORSTEN TONN ET AL: "Treatment of patients with advanced cancer with the natural killer cell line NK-92", CYTOTHERAPY, ISIS MEDICAL MEDIA, OXFORD, GB, vol. 15, no. 12, 1 December 2013 (2013-12-01), pages 1563-1570, XP002729819, ISSN: 1465-3249, DOI: 10.1016/J.JCYT.2013.06.017 [retrieved on 2013-10-01]

Remarks:
The file contains technical information submitted after the application was filed and not included in this specification

**Description**

Technical Field

The present invention relates to a method for producing cryopreserved NK cells and a method of producing NK cells from frozen CD3-negative cells.

Background Art

For tumor treatment, various treatment methods, including surgery, radiotherapy and chemotherapy, have been developed. However, in the case of some tumors, frequent recurrence of the tumors has posed a serious problem. For this reason, the potential of cellular therapy based on patient's immunity has been proposed.

Immune responses that remove tumors are caused by complex interactions between immune cells having various functions. Immune cells that directly remove tumor cells include natural killer cells (hereinafter referred to as NK cells) and cytotoxic T lymphocytes (CTLs), and antigen-presenting cells that present antigens to these effector cells include dendritic cells (DCs) and B cells. In addition to these cells, there are helper T cells, regulatory T cells and the like, which release various cytokines.

Among the cells of the immune cells, NK cells are a type of lymphocytes and are distributed in human bone marrow, spleen, peripheral lymph nodes and peripheral blood, and it is known that about 10% of lymphocytes in the peripheral blood are NK cells (Ann Rev Immunol., 24: 257-286, 2006). NK cells are positive for CD56 and CD16 but negative for CD3. Unlike T-cells, NK cells kill tumor cells and virally infected cells without previous stimulation or MHC restriction, and do not express clonally rearranged receptors (Trends Immunol., 22: 633-640, 2001). NK cell-mediated apoptosis is associated with the release of cytoplasmicc granules containing perforin and granzyme and pathways including FasL and TRAIL. NK cells release various cytokines, particularly IFN-$\gamma$, INF-$\alpha$, GM-CSF and IL-10, and express various receptors on the cell surface, and these receptors are involved in cell adhesion, activation of cytotoxicity, or inhibition of cytotoxicity. Furthermore, NK cells recognize MHC class I molecules via KIRs (killer immunoglobulin-like receptors), and most KIRs are killing inhibitory receptors. When such inhibitory receptors are not recognized as MHC molecules, cell killing will occur.

Based on this cytotoxicity of NK cells, new cellular therapies have been attempted either to treat solid tumors using lymphokine activated killer cells (LAK) and tumor infiltration lymphocytes (TILs) or to perform immunotherapy through donor lymphocyte infusion (Tilden. A.B. et al, J. Immunol., 136: 3910-3915, 1986; Bordignon C. et al., Hematologia, 84: 1110-1149, 1999) to thereby prevent rejection from occurring in bone marrow implantation or organ transplantation. In addition, it was reported that the defect in NK cell differentiation and activity is related to various cancer diseases, including breast cancer (Konjevic G. et al., Breast Cancer Res. Treat., 66: 255-263, 2001), melanoma (Ryuke Y. et al., Melanoma Res., 13: 349-356, 2003), and lung cancer (Villegas FR., et al., Lung Cancer, 35: 23-28, 2002). Thus, to treat such diseases, NK cell therapy is attracting attention.

Most NK cells present *in vivo* in a normal state are present in an inactivated state. However, to use NK cells for actual therapeutic applications, activated NK cells are required. For this reason, studies on activation of NK cells from normal blood or inactivated patient's blood have been actively conducted.

High NK cell cytotoxicity achieved by *in vitro* activation of NK cells demonstrated the cellular immunotherapy potential of NK cells. It was reported that NK cells activated *in vitro* exhibit therapeutic effects against various types of cancer, particularly blood cancer such as leukemia, when they are administered after allogenic bone marrow transplantation (Blood Cells Molecules & Disease, 33: 261-266, 2004). However, the clinically distinct therapeutic effect of NK cells against solid cancers other than blood cancer has not yet been demonstrated. Specifically, it was reported that administration of NK cells before development of a tumor can interfere with engraftment of the tumor (Cancer Immunol. Immunother., 56(11): 1733-1742, 2007), but it hardly appears to be a suitable therapeutic model. In addition, there are animal study results indicating that intraperitoneal administration of NK cells inhibited the growth of breast cancer cells, but it is unclear whether this effect results from NK cells (Breast Cancer Res. Treat., 104(3): 267-275, 2007).

In addition, in order to effectively use NK cells for anticancer cellular immunotherapy, it is required to obtain a large number of NK cells. However, because NK cells account for 10-15% of lymphocytes in blood and the number, differentiation and function of NK cells in cancer patients are often reduced, it is difficult to actually obtain a sufficient number of NK cells. Accordingly, it is urgently required to obtain a large amount of NK cells by proliferation or differentiation of NK cells.

It is known that NK cells are derived from hematopoietic stem cells (HSCs). Methods for inducing differentiation into NK cells were reported which comprise isolating hematopoietic stem cells from umbilical cord blood *in vitro* and treating and culturing the isolated cells with suitable cytokines to thereby induce differentiation into NK cells (Galy et al., Immunity 3: 459-473, 1995; Mrozek E, et al., Blood 87:2632-2640, 1996; Sivori, S. et al., Eur J Immunol. 33:3439-3447, 2003; B. Grzywacz, et al., Blood 108: 3824-3833, 2006). Specifically, these methods may comprise adding Flt-3L, IL-7, SCF and IL-15 to CD34[+] HSCs and culturing the HSCs for 5 weeks to induce differentiation into CD3[-]CD56[+] NK cells. However, such differentiation methods have shortcomings in that it is difficult to obtain a sufficient amount of cells for treatment and in that much time and cost are required, indicating that these methods are difficult to apply to actual clinical practice.

It is known that, in mice deficient in expression of $\gamma_c$ of cytokine receptors, B cells and T cells are found but NK cells are not found, indicating that receptors having $\gamma_c$ play an important role in NK cell differentiation (Singer, B et al., Proc. Natl. Acad. Sci. USA 92, 377-381, 1995). The $\gamma_c$ forms of receptor include IL-2, IL-4, IL-7, IL-9, IL-15 and IL-21 receptors, and among them, IL-2 was reported to function to promote the proliferation and activation of mature NK cells (Shibuya, A. et al., Blood 85, 3538-3546, 1995). It was reported that the number of NK cells in IL-2-deficient humans and mice significantly decreases (DiSanto, J. P. et al., J. Exp. Med. 171, 1697-1704, 1990), but there are also study results indicating that deficiency of IL-2 and IL-2Ra has an indirect effect on the number and activation of NK cells. In addition, IL-2R (IL-2 receptor) chains are involved in formation of IL-15 receptor.

IL-15 is involved in NK cell differentiation, as demonstrated by the fact that mice lacking interferon-regulating factor-1 (IRF-1) that is transcription factor required for IL-15 production lack NK cells (Kouetsu et al., Nature 391, 700-703, 1998) and that NK cells are not found in mice lacking IL-15 or IL-15Ra. Thus, it was reported that IL-15 directly promotes the growth and differentiation of NK cells by the IL-15 receptor that is expressed in NK cells (MrozekE et al., Blood 87, 2632-2640, 1996).

IL-21 is a cytokine which is secreted by activated CD4[+]T cells (Nature, 5:688-697, 2005), and the IL-21 receptor (IL-21R) is expressed in lymphocytes such as dendritic cells, NK cells, T cells and B cells (Rayna Takaki, et al., J. Immonol 175: 2167- 2173, 2005). IL-21 is structurally very similar to IL-2 and IL-15, and IL-21R shares a chain with IL-2R, IL-15, IL-7R and IL-4R (Asao et al., J. Immunol, 167: 1-5, 2001). It was reported that IL-21 induces the maturation of NK cell progenitors from bone marrow (Parrish-Novak, et al., Nature, 408: 57-63, 2000). Particularly, it was reported that IL-21 increases the effector functions (such as cytokine-producing ability and apoptotic ability) of NK cells (M. Strengell, et al., J Immunol, 170: 5464-5469, 2003; J. Brady, et al., J Immunol, 172: 2048-2058, 2004), and that IL-21 also increases the effector functions of CD8[+]T cells, thereby promoting the anticancer responses of innate and adaptive immune systems (Rayna Takaki, et al., J Immunol 175: 2167-2173, 2005; A. Moroz, et al., J Immunol, 173: 900-909, 2004). Furthermore, it was reported that IL-21 activates NK cells isolated from human peripheral blood (Parrish-Novak, et al., Nature, 408: 57, 2000), and plays an important role in inducing mature NK cells from hematopoietic stem cells isolated from umbilical cord blood (J. Brady, et al., J Immunol, 172: 2048, 2004).

KR20140051263A discloses a method for producing an optimum number of NK cells for a cell therapy by amplifying the NK cells from collected blood cells, including the steps of cultivating in the culture medium and removing the CD3 positive cells.

Meanwhile, despite the potential of the above-described cells as cancer therapeutic agents, the number of NK cells present *in vivo* is not large. For this reason, in order to use such NK cells as cancer therapeutic agents, a technology is necessarily required which produces NK cells in large amounts enabling the sufficient efficacy of NK cells to be maintained *in vivo*. However, there is a problem in that *in vitro* proliferation and culture of a large amount of NK cells are not properly achieved. For this reason, it has been required to develop a technology for culturing and proliferating NK cells at an actually useful level, and many studies on this development have been conducted. However, a clinically applicable level has not yet been achieved.

Disclosure

Technical Problem

Accordingly, the present inventors have conducted studies to develop a method for producing a large amount of NK cells in a more efficient and economical manner. As a result, the present inventors have found that, when CD3-negative cells obtained by removing CD3-positive T cells from monocytes are treated with cytokines such as IL-15 and IL-21 and then cultured, a large amount of highly pure NK cells can be produced within a short time compared to conventional NK cell production methods, and that fresh NK cells produced by this method of the present invention inhibit the growth of cancer cells in mouse models xenografted with colorectal cancer, lung cancer, liver cancer and pancreatic cancer cell lines, reduce the weight of cancer cells in the mouse models, and also exhibit their therapeutic effects in blood cancer such as leukemia, indicating that the NK cells produced by the method of the present invention can be used as a pharmaceutical composition for preventing or treating cancer. Furthermore, the present inventors have identified the dose of NK cells and a method for administration of NK cells in treatment of actual cancer patients with NK cells produced by the method of the present invention.

In addition, the present inventors have found that cold-preserved NK cells and cryopreserved NK cells exhibit anticancer effects comparable with fresh NK cells depending on conditions where fresh NK cells are cold-preserved or cryopreserved, and have also identified the dose of NK cells and a method for administration of NK cells in each of the case in which cold-preserved NK cells are used alone, the case in which cryopreserved NK cells are used alone, and the case in which cold-preserved NK cells or cryopreserved NK cells are used in a mixture with fresh NK cells.

In addition, the present inventors have found that fresh NK cells can be produced by thawing cryopreserved CD3-negative cells depending on conditions where CD3-negative cells are cryopreserved.

Technical Solution

The present disclosure provides a method for producing fresh NK cells, the method comprising the steps of:

1) removing CD3-positive T cells from monocytes to obtain CD3-negative cells; and
2) culturing the CD3-negative cells by treating the CD3-negative cells of step 1) with IL-15 and IL-21,

wherein step 1) is performed by allowing the CD3-positive T cells to crosslink to erythrocytes and then isolating the CD3-negative cells by density-gradient centrifugation.

In the present disclosure, the isolation of the CD3-negative cells by density-gradient centrifugation by allowing the CD3-positive T cells to crosslink to erythrocytes in step 1) may be performed using an antibody that uses the CD3-negative cells. For example, the isolation of the CD3-negative cells may be performed using the product RosetteSep™ Human NK Cell Enrichment Cocktail (manufactured and marketed by STEMCELL Technologies Inc.). The above produce is a cocktail of tetrameric antibody complexes recognizing CD3, CD4, CD19, CD36, CD66b, CD123 and glycophorin A. CD3 positive cells, CD4 positive cells, CD19 positive cells, CD36 positive cells, CD66b positive cells and CD123 positive cells bind to the tetrameric antibody complexes together with erythrocytes expressing glycophorin A to form cross-links. When centrifugation is then performed, the cells form a pellet by density gradient, and a medium comprising a high concentration of CD3-negative NK cells can be obtained from the upper layer portion.

In an embodiment of the present disclosure, the culturing in step 2) may be performed at a cell concentration of $1 \times 10^6$ cells/ml, but this cell concentration may be properly determined by those skilled in the art such that it causes no abnormalities in the morphology and activity of the cells.

In one embodiment of the present disclosure, the culturing in step 2) may be performed for 10-24 days, but the culture period may be determined by confirming that the cultured cells show a characteristic of $CD3^-CD56^+$.

In one embodiment of the present disclosure, the culturing in step 2) may be performed at a cell concentration of $1 \times 10^6$ cells/ml.

In one embodiment of the present disclosure, the culturing in step 2) may be performed for 10-24 days.

In the method, the culturing in step 2) may be performed using a stationary culture or suspension culture method. As used herein, the term "stationary culture" means that cells are cultured in an incubator without agitating or shaking, and the term "suspension culture" means that cells are cultured in a suspended state by aeration or agitation such that the cells are not attached to the bottom or side portion of the reactor. Furthermore, the reactor for stationary culture and the reactor for suspension culture may be the same or different. For example, when the reactor for stationary culture and the reactor for suspension culture are the same, stationary culture is completed in the same reactor and then a medium containing necessary nutrient components such as cytokine may additionally be supplied to the same reactor, followed by suspension culture. When different reactors are used, the cultured cells after stationary culture may be transferred into the reactor for suspension culture.

In the present disclosure, the fresh NK cells may be $CD3^-$ $CD56^+$.

In the present disclosure, the NK cells are preferably derived from umbilical cord blood, bone marrow or peripheral blood monocytes, but any type of cells may be used as progenitor, as long as they are CD3-negative cells.

The present disclosure provides a pharmaceutical composition for preventing or treating cancer comprising the fresh NK cells produced by the above-described method, as an active ingredient.

In the present disclosure, the pharmaceutical composition means a "cellular therapeutic agent". As used herein, the term "cellular therapeutic agent" refers to cells and tissues prepared by isolation from an individual, culture and special operations, and means a pharmaceutical product (US FDA regulations) which is used for the purposes of treatment, diagnosis and prevention and which is obtained through a series of actions, including growing and screening living autologous or allogenic cells *in vitro* in order to restore the structure and function of the cells or changing the biological characteristics of cells by any other methods.

In the present disclosure, the cancer is not limited as long as it is a cancer that can be treated with NK cells. For example, the cancer may be any one selected from the group consisting of liver cancer, lung cancer, colorectal cancer, breast cancer, prostate cancer, ovarian cancer, pancreatic cancer, cervical cancer, thyroid cancer, laryngeal cancer, leukemia, brain tumor, neuroblastoma, retinoblastoma, head and neck cancer, salivary gland cancer, and lymphoma. Preferably, the cancer may be any one cancer selected from the group consisting of colorectal cancer, lung cancer, liver cancer, pancreatic cancer, and leukemia.

In one embodiment of the present disclosure, the composition may comprise $1 \times 10^5$ or more, $3 \times 10^5$ or more,

$3 \times 10^6$ or more, $1 \times 10^6$ or more, $3 \times 10^6$ or more, $6 \times 10^6$ or more, or $1 \times 10^7$ or more fresh NK cells.

In one embodiment of the present disclosure, the composition may further comprise IL-2.

In the present disclosure, the composition may be administered at intervals of 14-42 days, preferably 14-35 days, more preferably 14-30 days. However, the interval of administration is not limited thereto.

In one embodiment of the present disclosure, the composition may be administered once a week for 4 weeks, or may be administered twice a week for 2 weeks.

In one embodiment of the present disclosure, the composition may comprise $3 \times 10^6$ fresh NK cells and may be administered once a week for 4 weeks.

In another embodiment of the present disclosure, the composition may comprise $3 \times 10^6$ fresh NK cells and may be administered twice a week for 2 weeks.

In another embodiment of the present disclosure, the composition may comprise $6 \times 10^6$ fresh NK cells and may be administered once a week for 2 weeks.

The present disclosure also provides a method for producing cold-preserved NK cells, the method comprising the steps of:

1) removing CD3-positive T cells from monocytes to obtain CD3-negative cells;
2) culturing the CD3-negative cells by treating the CD3-negative cells of step 1) with IL-15 and IL-21; and
3) preserving the CD3-negative cells at 4°C for 15 hours or less,

wherein step 1) is performed by allowing the CD3-positive T cells to crosslink to erythrocytes and then isolating the CD3-negative cells by density-gradient centrifugation.

In the present disclosure, the CD3-negative cells in step 3) may be preserved for 15 hours or less, for example, 12 hours or more, for example, 10 hours or less. However, the preservation time is not limited thereto.

In the present disclosure, the cold-preserved NK cells may be CD3⁻CD56⁺.

The present disclosure also provides a pharmaceutical composition for preventing or treating cancer comprising cold-preserved NK cells produced by the above-described method, as an active ingredient.

In the present disclosure, the cancer may be any one cancer selected from the group consisting of colorectal cancer, lung cancer, liver cancer, pancreatic cancer, and leukemia. However, the kind of cancer is not limited thereto, and may be any cancer that can be prevented, alleviated or treated with NK cells.

In one embodiment of the present disclosure, the composition may comprise $1 \times 10^5$ or more, $3 \times 10^5$ or more, $3 \times 10^6$ or more, $1 \times 10^6$ or more, $3 \times 10^6$ or more, $6 \times 10^6$ or more, or $1 \times 10^7$ or more cold-preserved NK cells.

In one embodiment of the present disclosure, the composition may further comprise IL-2.

In the present disclosure, the composition may be administered at intervals of 14-42 days, preferably 14-35 days, more preferably 14-30 days. However, the interval of administration is not limited thereto.

In one embodiment of the present disclosure, the composition may be administered once a week for 4 weeks, or may be administered twice a week for 2 weeks.

In one embodiment of the present disclosure, the composition may comprise $3 \times 10^6$ cold-preserved NK cells and may be administered once a week for 4 weeks.

In another embodiment of the present disclosure, the composition may comprise $3 \times 10^6$ cold-preserved NK cells and may administered twice a week for 2 weeks.

In another embodiment of the present disclosure, the composition may comprise $6 \times 10^6$ cold-preserved NK cells and may administered once a week for 2 weeks.

The present invention also provides a method for producing cryopreserved NK cells, the method comprising the steps of:

1) removing CD3-positive T cells from monocytes to obtain CD3-negative cells;
2) culturing the CD3-negative cells by treating the CD3-negative cells of step 1) with IL-15 and IL-21; and
3) freezing the cultured CD3-negative cells of step 2) in a cryopreservation medium containing 10% DMSO (dimethyl sulfoxide) under serum-free, protein-free and animal component-free conditions for 2 months or less, wherein the freezing is performed by stepwise cooling from -70°C to -200°C.

As used herein, the term "freezing" means an operation of freezing the NK cells of the present invention. The freezing may be performed using various freezing media. For example, the freezing may be performed using a cryopreservation box containing isopropyl alcohol. However, the freezing may also be performed using other means. In addition, in order to prevent damage to the cells during the freezing, a solution containing a cryoprotective agent may be used. As used herein, the term "cryoprotective agent" refers to a substance which is added to a medium for the purpose of reducing frost damage when living biological cells are preserved in a frozen state. The cryoprotective agent that may be used in the present invention may be glycerol, sugar, glucose or the like, but is not particularly limited thereto.

In one embodiment of the present invention, the freezing period may be performed for 2 months or less, preferably 6 weeks or less, more preferably 1 month or less. However, the freezing period is not limited thereto, and may be suitably adjusted within a range in which the effect of the cryopreserved NK cells is maintained.

In one embodiment of the present disclosure, the concentration of the CD3-negative cells in step 3) may be $1.5 \times 10^7$ cells/ml, but is not limited thereto.

The present disclosure also provides a method for producing thawed cryopreserved NK cells, the method comprising the steps of:

A method for producing thawed cryopreserved NK cells, the method comprising the steps of:

1) removing CD3-positive T cells from monocytes to obtain CD3-negative cells;
2) culturing the CD3-negative cells by treating the CD3-negative cells of step 1) with IL-15 and IL-21;
3) freezing the cultured CD3-negative cells of step 2) in a cryopreservation medium containing 10% DMSO (dimethyl sulfoxide) under serum-free, protein-free and animal component-free conditions for 2 months or less, wherein the freezing is performed by stepwise cooling from -70°C to -200°C; and
4) quick thawing the cryopreserved NK cells at 37°C, and washing out the cryopreservation medium.

As used herein, the term "thawing" refers to an operation that increases the temperature of the cryopreserved NK cells to room temperature so as to enable the cells to exhibit normal physiological activity during use.

In the present invention, the thawed cryopreserved NK cells may be CD3⁻.

The present disclosure also provides a pharmaceutical composition for preventing or treating cancer comprise thawed cryopreserved NK cells produced by the above-described method, as an active ingredient.

In the present disclosure, the cancer may be any one cancer selected from the group consisting of colorectal cancer, lung cancer, liver cancer, pancreatic cancer, and leukemia. However, the kind of cancer is not limited thereto, and may be any cancer which can be prevented, alleviated or treated with NK cells.

In one embodiment of the present disclosure, the composition may comprise $1 \times 10^5$ or more, $3 \times 10^5$ or more, $3 \times 10^6$ or more, $1 \times 10^6$ or more, $3 \times 10^6$, $6 \times 10^6$ or more, or $1 \times 10^7$ or more thawed cryopreserved NK cells.

In one embodiment of the present disclosure, the composition may further comprise distilled water or serum-free medium.

In one embodiment of the present disclosure, the composition may further comprise IL-2.

In the present disclosure, the composition may be administered at intervals of 14-42 days, preferably 14-35 days, more preferably 14-30 days. However, the administration interval is not limited thereto.

In one embodiment of the present disclosure, the composition may be administered once a week for 4 weeks, or may be administered twice a week for 2 weeks, or may be administered twice a week for 4 weeks.

In one embodiment of the present disclosure, the composition may comprise $3 \times 10^6$ NK thawed cryopreserved cells, and may be administered once a week for 4 weeks.

In another embodiment of the present disclosure, the composition may comprise $3 \times 10^6$ NK thawed cryopreserved cells, and may be administered twice a week for 2 weeks.

In another embodiment of the present disclosure, the composition may comprise $6 \times 10^6$ thawed cryopreserved NK cells, and may be administered once a week for 2 weeks.

The present invention also provides a method of producing NK cells from frozen CD3-negative cells, the method comprising the steps of:

1) removing CD3-positive T cells from monocytes to obtain CD3-negative cells;
2) freezing the obtained CD3-negative cells of step 1) in a cryopreservation medium containing 10% DMSO (dimethyl sulfoxide) under serum-free, protein-free and animal component-free conditions for 2 months or less, wherein the freezing is performed by stepwise cooling from -70°C to -200°C;
3) thawing the frozen CD3-negative cells of step 2); and
4) culturing the thawed CD3-negative cells by treating the thawed CD3-negative cells of step 3) with IL-15 and IL-21.

In the present invention, the NK cells produced from frozen CD3-negative cells by the above-described method have the same properties and effects as those of fresh NK cells. Thus, the NK cells produced from frozen CD3-negative cells may be used as an active ingredient in a composition for preventing or treating cancer, in the same manner as fresh NK cells.

The present disclosure also provides a pharmaceutical composition for preventing or treating cancer comprising the fresh NK cells and thawed NK cells produced according to the above-described methods of the present invention, as an active ingredient.

In one embodiment of the present disclosure, each of the fresh NK cell and the thawed NK cells may be comprised in a single dose in an amount of $1 \times 10^5$ or more cells, $3 \times 10^5$ or more cells, $3 \times 10^6$ or more cells, $1 \times 10^6$

or more cells, $3 \times 10^6$ or more cells, $6 \times 10^6$ or more cells, or $1 \times 10^7$ or more cells.

In one embodiment of the present disclosure, the fresh NK cell and the thawed NK cells may be administered once a week for 4 weeks, or administered twice a week for 2 weeks, or administered twice a week for 4 weeks. Preferably, the fresh NK cells may be administered once a week for 1 week, and the thawed cryopreserved NK cells may be administered twice a week for 3 weeks. However, the administration method may be suitably adjusted.

The pharmaceutical composition of the present disclosure may be prepared using a pharmaceutically suitable and physiologically acceptable adjuvant in addition to the active ingredient. The adjuvant may be one or more of an excipient, a disintegrating agent, a sweetener, a binder, a coating agent, a swelling agent, a lubricant and a flavoring agent.

For administration, the composition of the present disclosure may be formulated to comprise one or more pharmaceutically acceptable carriers in addition to the above-described active ingredient. The pharmaceutically acceptable carriers include saline, sterile water, Ringer's solution, buffered saline, dextrose solution, malto-dextrin solution, glycerol, ethanol, liposome and a mixture of one or more of these components. If necessary, the composition of the present disclosure may comprise other conventional additives, including antioxidants, buffers, and bacteriostatic agents. In addition, a diluent, a dispersing agent, a surfactant, a binder and a lubricant may further be added to the composition of the present disclosure to thereby prepare an injectable formulation such as an aqueous solution, a suspension or an emulsion, or a pill, capsule, granule or tablet formulation. Furthermore, a target organ-specific antibody or ligand bound to the carrier may be used so that the composition can act specifically in the target organ. Furthermore, the composition of the present disclosure may preferably formulated by a suitable method known in the art or a method disclosed in Remington's Pharmaceutical Science (the latest edition, Mack Publishing Company, Easton PA) to prepare formulations suitable for each disease or component.

The pharmaceutical composition of the present disclosure may be provided as a liquid, suspension, dispersion, emulsion, gel, injectable solution or sustained-release formulation of the active ingredient. Preferably, the composition of the present disclosure may be formulated as an injectable solution.

If the pharmaceutical composition of the present disclosure is formulated as an injectable solution, it may be prepared as a physically or chemically very stable injectable solution by adjusting the pH with an aqueous acid solution or a buffer such as phosphate, which may be used for injection, in order to ensure the stability of the injectable formulation during distribution.

More specifically, the injectable formulation may be prepared by dissolving the composition in injectable water together with a stabilizer or a dissolution aid, and then sterilizing the solution by high-temperature sterilization under reduced pressure or by sterile filtration. The injectable water may be injectable distilled water or an injectable buffer, for example, phosphate buffered saline (pH 3.5 to 7.5) or sodium dihydrogen phosphate ($NaH_2PO_4$)-citrate buffer. The phosphate used may be in the form of sodium salt, potassium salt, anhydride or hydrate, and may also be in the form of citrate, anhydride or hydrate.

Furthermore, the stabilizer that is used in the present disclosure comprises sodium pyrosulfite, sodium bisulfite ($NaHSO_3$), sodium metabisulfite ($Na_2S_2O_3$) or ethylenediaminetetraacetic acid, and the dissolution aid comprises a base such as sodium hydroxide (NaOH), sodium hydrogen carbonate ($NaHCO_3$), sodium carbonate ($NaCO_3$) or potassium hydroxide (KOH), or an acid such as hydrochloric acid (HCl) or acetic acid ($CH_3COOH$).

The injectable formulation according to the present disclosure can be prepared to be bioabsorbable, biodegradable, biocompatible. "Bioabsorbable" means that the injectable formulation is capable of disappearing from its initial application site in the body, with or without degradation of the dispersed injectable formulation. "Biodegradable" means that the injectable formulation is capable of breaking down or degrading within the body, by hydrolysis or enzymatic degradation. Biocompatible means that all of the components are nontoxic in the body.

The injectable formulation according to the present disclosure may be prepared using conventional diluents including fillers, extenders, binders, wetting agents and surfactants or excipients.

The composition or active ingredient of the present disclosure may be administered using a conventional method by an intravenous, intra-arterial, intraperitoneal, intramuscular, intrasternal, transdermal, intranasal, subcutaneous, intrauterine, inhalation, topical, intrarectal, oral, intraocular or intradermal route depending on the intended use. Preferably, it may be administered intravenously. The composition or active ingredient of the present disclosure may be administered by injection or catheter.

In the composition of the present disclosure, the dose of the active ingredient may be adjusted within the range of $1 \times 10$ to $1 \times 10^{50}$ cells/kg, preferably $1 \times 10$ to $1 \times 10^{30}$ cells/kg, more preferably $1 \times 10^5$ to $1 \times 10^{20}$ cells/kg, most preferably $1 \times 10^7$ to $1 \times 10^9$ cells/ kg, for an adult weighing 60 kg. However, the optimal dose can be easily determined by those skilled in the art, and may vary depending on various factors, including the kind of disease, the severity of the disease, the contents of the active ingredient and other components in the composition, the type of the formulation, and the patient's age, body weight, general health condition, sex and diet, the time of administration, the route of administration, the secretion rate of the composition, the time period of treatment, and a particular drug which is used in combination with the composition.

In the composition of the present disclosure, the active ingredient may be contained in an amount of 0.001-50 wt% based on the total weight of the composition. However, the content of the active ingredient is not limited thereto.

The composition of the present disclosure may further contain one or more anticancer agents.

The present disclosure provides a method for preventing or treating cancer, comprising administering a therapeutically effective amount of the fresh NK cells to subjects in need of cancer treatment.

In the present disclosure, the subjects in need of cancer treatment may be mammals, including humans. For examples, the subjects may be humans, dogs, cats, horses or the like.

The present disclosure provides a method for preventing or treating cancer, comprising administering a therapeutically effective amount of the cryopreserved NK cells to subjects in need of cancer treatment.

The present disclosure provides a method for preventing or treating cancer, comprising administering a therapeutically effective amount of the thawed cryopreserved NK cells to subjects in need of cancer treatment.

As used herein, the term "therapeutically effective amount" refers to the amount of the active ingredient or pharmaceutical composition that evokes a biological or pharmaceutical response within an animal or human subject, and is an amount that is determined by researchers, veterinarians, doctors or other clinicians. The therapeutically effective amount includes an amount that leads to alleviation of symptoms of the disease or disorder to be treated. It is obvious to those skilled in the art that the therapeutically effective amount of the active ingredient and the number of administrations of the active ingredient according to the present disclosure can vary depending on the desired effect.

The present disclosure provides the use of the fresh NK cells for preparing a medicament for cancer treatment.

The present disclosure provides the use of the cold-preserved NK cells for preparing a medicament for cancer treatment.

The present disclosure provides the use of the thawed cryopreserved NK cells for preparing a medicament for cancer treatment.

Advantageous Effects

The use of the methods of the present invention can produce fresh NK cells with high purity within a short time compared to conventional method, and can also produce cold-preserved NK cells and thawed cryopreserved NK cells, which have efficacy comparable with that of the fresh NK cells. Furthermore, it can produce NK cells, which have efficacy comparable with that of the fresh NK cells, from cryopreserved CD3-negative cells.

The fresh NK cells, cold-preserved NK cells and cryopreserved NK cells produced by the methods of the present invention can exhibit therapeutic effects against various cancers, including colorectal cancer, lung cancer, liver cancer, pancreatic cancer and leukemia, indicating that these NK cells can be effectively used as cellular therapeutic agents.

In addition, the present inventors have established doses and methods of administration, which show excellent effects when the fresh NK cells, cold-preserved NK cells and cryopreserved NK cells of the present invention are used as pharmaceutical compositions for cellular therapy.

Description of Drawings

FIG. 1 shows FACS results indicating that differentiation of NK cells from CD3-negative cells isolated either from umbilical cord blood (the top of FIG. 1) or from peripheral blood (the bottom of FIG. 1) was induced after 11-21 days of culture.

FIG. 2a shows the viability of thawed NK cells obtained by cryopreserving fresh NK cells on 10 days of culture and thawing the cryopreserved NK cells; FIG. 2b shows the degree of differentiation; FIG. 2c shows NK cell receptors; and FIG. 2d show NK cell cytotoxicities.

FIG. 2e shows the cell recovery rate immediately after thawing of NK cells obtained by differentiation of CD3-negative cells thawed after freezing; FIG. 2f shows fold increase in cell number upon culture after thawing; FIG. 2g shows cell viability; and FIG. 2h shows the degree of differentiation and NK cell receptors.

FIG. 3a shows the results of detecting NK cells in mice to measure the concentration-dependent detection limit of NK (natural killer) cells.

V.C (5% HSA): vehicle control group.

FIG. 3b shows the results of detecting NK cells in the major intra-abdominal organs of mice to mesure the concentration-dependent detection limit of NK cells.

V.C (5% HSA): vehicle control group.

FIG. 3c shows the results of analyzing the distribution of NK cells in the major intra-abdominal organs of mice.

V.C: vehicle control group.

FIG. 3d shows the results of analyzing the *in vivo* distribution of NK cells in mice at varying time points.

FIG. 4a shows an administration schedule for examining the anticancer effect of NK cells against colorectal cancer.

FIG. 4b shows the results of measuring the tumor size inhibitory effect of NK cells alone or in combination with IL-2 against colorectal cancer when varying number of the NK cells are used.

V.C: vehicle control group, and

ADR: adriamycin-treated group.

FIG. 4c shows the results of measuring the tumor weight reducing effect of NK cells alone or in combination with IL-2 against colorectal cancer when varying number of the NK cells are used.

V.C: vehicle control group, and

ADR: adriamycin-treated group.

FIG. 5a shows administration schedules prepared to examine anticancer effects according to the culture conditions, preservation conditions and administration schedule of NK cells.

FIG. 5b shows the results of analyzing the tumor volume inhibitory effects of NK cells against colorectal cancer according to the culture conditions, preservation conditions and administration schedule of NK cells.

V.C: vehicle control group;

NK-F: fresh NK cell-treated group;

NK-W/oR-F: group treated with fresh NK cells (Rosettesep-free, that is, w/o Rosettesep);

NK-4°C: group treated with NK cells cold-preserved at 4°C;

NK-W/oR-4°C: group treated with fresh NK cells (Rosettesep-free) cold-preserved at 4°C;

ADR: adriamycin-treated group.

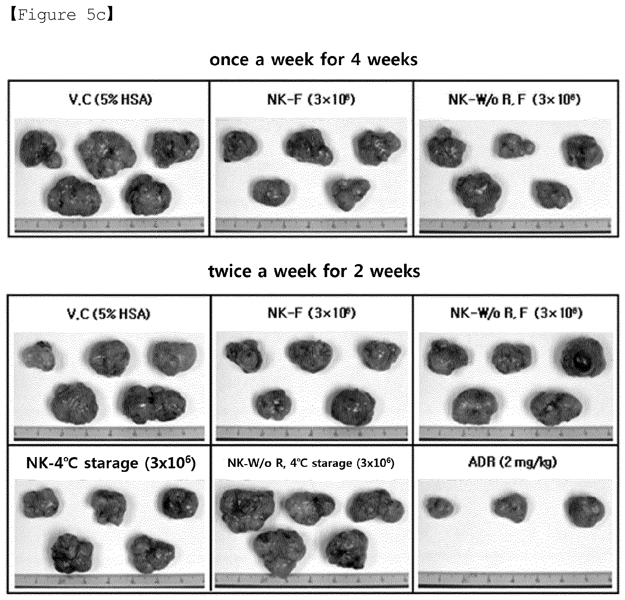

FIG. 5c shows the results of analyzing the tumor weight-reducing effects of NK cells against colorectal cancer according to the culture conditions, preservation conditions and administration schedule of NK cells.

V.C: vehicle control group;

NK-F: group treated with fresh NK cells;

NK-W/oR,F: group treated with fresh NK cells (Rosettesep-free);

NK-4°C preserved: group treated with NK cells cold-preserved at 4°C;

NK-W/oR, 4°C preserved: group treated with fresh NK cells (Rosettesep-free) cold-preserved at 4°C;

ADR: adriamycin-treated group.

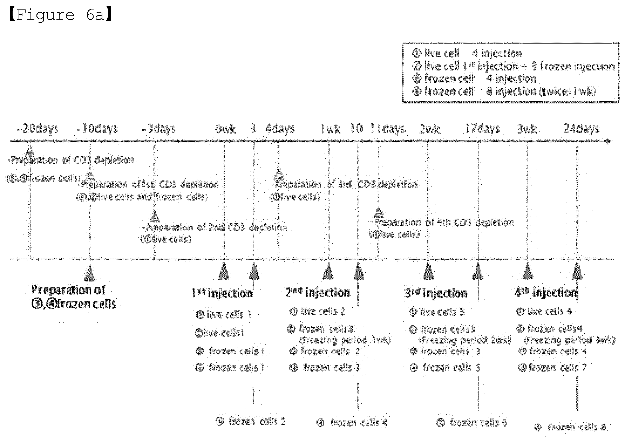

FIG. 6a shows an administration schedule prepared to examine the anticancer effect of NK cells according to freezing or not of NK cells.

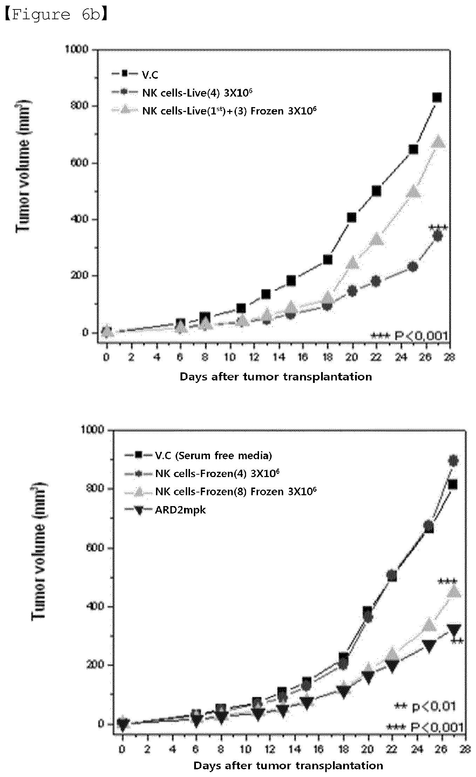

FIG. 6b shows the tumor volume inhibitory effect of NK cells against colorectal cancer according to freezing or not of NK cells.

V.C: vehicle control group;

NK cell-Live (4): group administered four times with fresh NK cells;

NK cell-Live (1)+(3) frozen: group administered once with fresh NK cells and three times with thawed cryopreserved NK cells;

V.C (serum-free medium): serum-free medium group;

NK cell-frozen (4): group administered four times with thawed cryopreserved NK cells;

NK cell-frozen (8): group administered eight times with thawed cryopreserved NK cells; and

ADR: adriamycin-treated group.

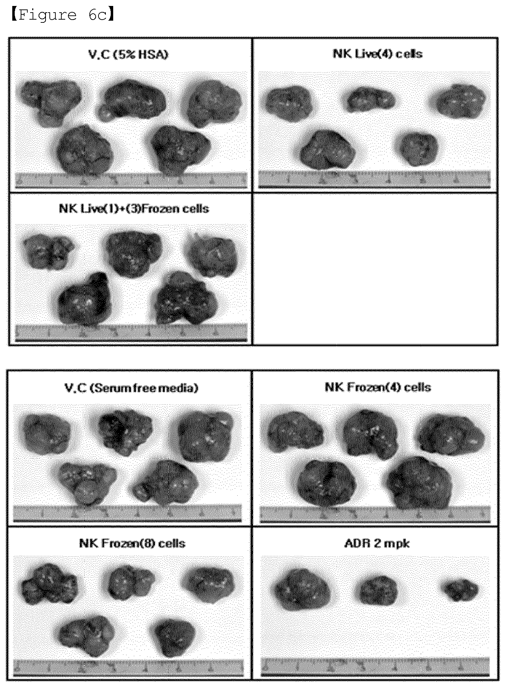

FIG. 6c shows the tumor weight-reducing effect of NK cells against colorectal cancer according to freezing or not of NK cells.

V.C: vehicle control group;

NK cell-Live (4): group administered four times with fresh NK cells;

NK cell-Live (1)+(3) frozen: group administered once with fresh NK cells and three times with thawed cryopreserved NK cells;

V.C (serum-free medium): serum-free medium group;

NK cell-frozen (4): group administered four times with thawed cryopreserved NK cells;

NK cell-frozen (8): group administered eight times with thawed cryopreserved NK cells; and

ADR 2 mpk: adriamycin-treated group.

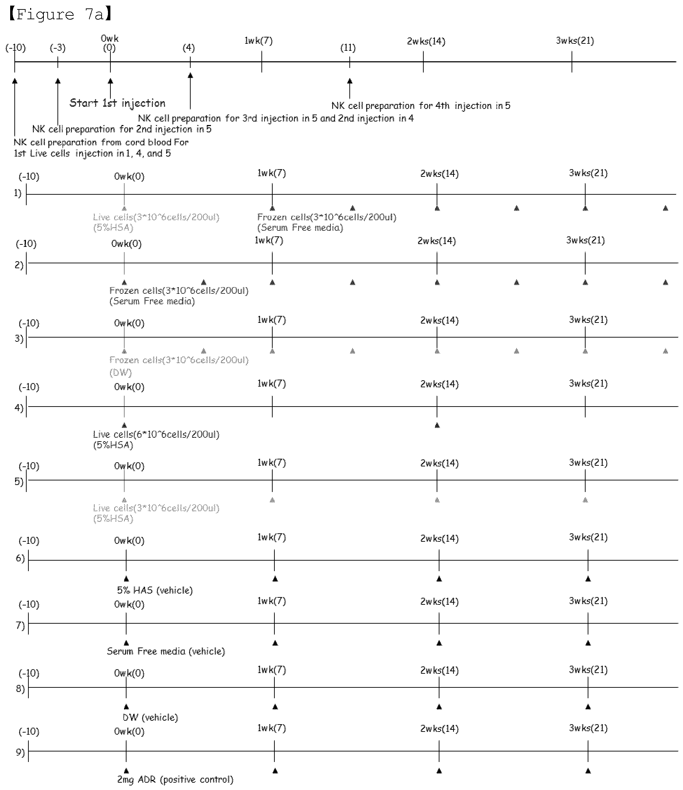

FIG. 7a shows administration schedules prepared to examine the anticancer effects of NK cells according to freezing or not of NK cells, the number of the NK cells and the number of administrations of the NK cells.

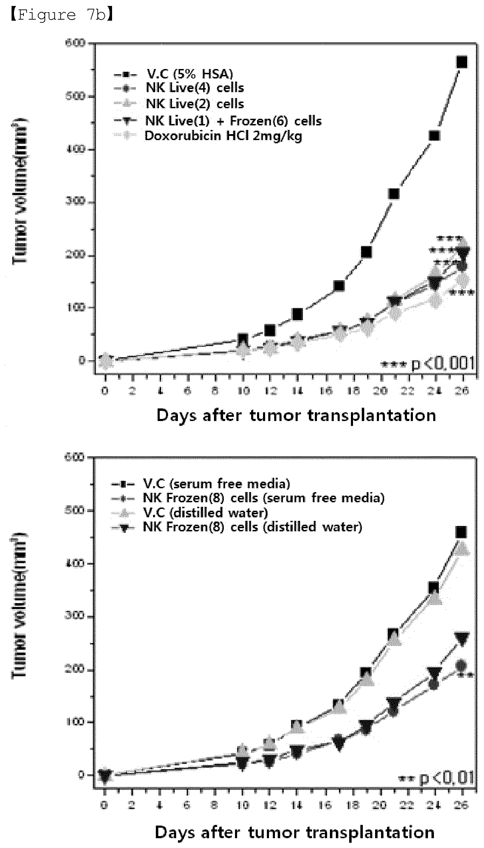

FIG. 7b shows the tumor volume effects of NK cells against colorectal cancer according to freezing or not of NK cells, the number of the NK cells and the number of administrations of the NK cells.

V.C (5% HSA): vehicle control group;

NK fresh (4) cells: group administered four times with fresh NK cells for 4 weeks;

NK fresh (2) cells: group administered twice with fresh NK cells for 4 weeks;

NK fresh (1)+(6) frozen cells: group administered once with fresh NK cells and then administered six times with thawed cryopreserved cells;

Doxorubicin HCL;

V.C (serum-free medium): serum-free control group;

NK frozen (8) cells (serum-free medium): group administered eight times with thawed cryopreserved NK cells in serum-free medium;

V.C (distilled water): sterile distilled water control group;

NK frozen (8) cells (distilled water): group administered eight times with thawed cryopreserved NK cells in sterile distilled water.

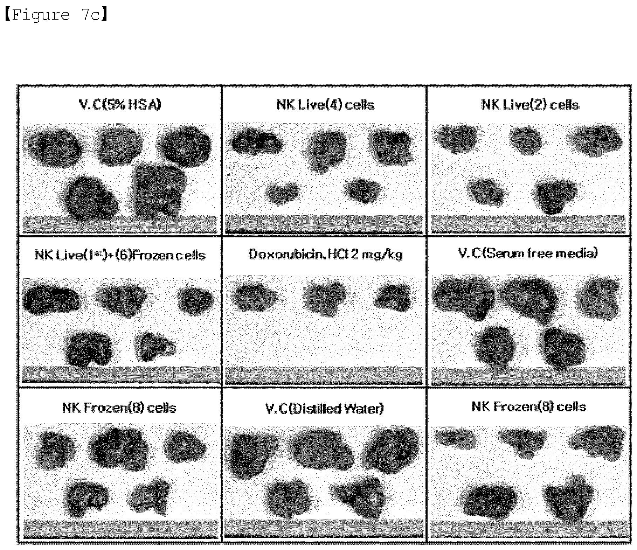

FIG. 7c shows the tumor weight-reducing effects of NK cells against colorectal cancer according to freezing or not of NK cells, the number of the NK cells and the number of administrations of the NK cells.

V.C (5% HSA): vehicle control group;

NK fresh (4) cells: group administered four times with fresh NK cells for 4 weeks;

NK fresh (2) cells: group administered twice with fresh NK cells for 4 weeks;

NK fresh (1)+(6) frozen cells: group administered once with fresh NK cells and then administered six times with thawed cryopreserved cells;

Doxorubicin HCL;

V.C (serum-free medium): serum-free control group;

NK frozen (8) cells (serum-free medium): group administered eight times with thawed cryopreserved NK cells in serum-free medium;

V.C (distilled water): sterile distilled water control group;

NK frozen (8) cells (distilled water): group administered eight times with thawed cryopreserved NK cells in sterile distilled water.

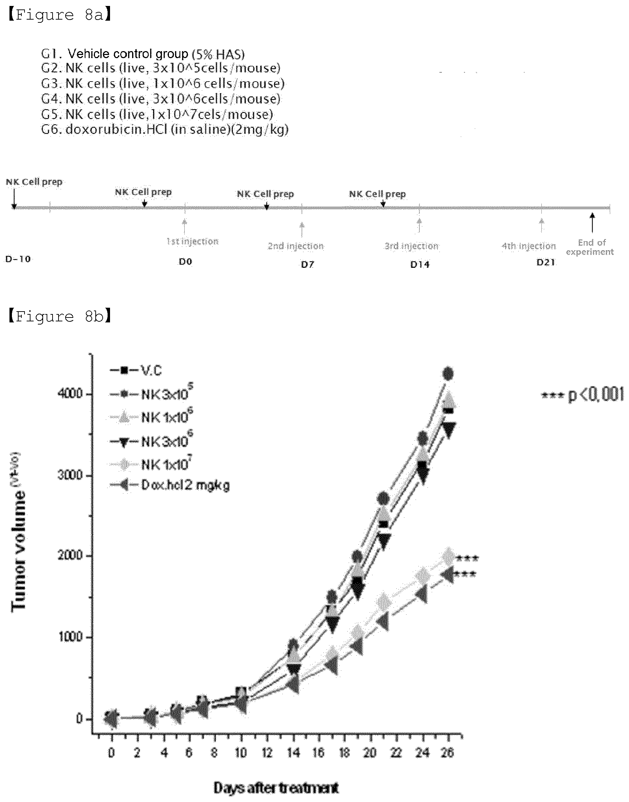

FIG. 8a shows an administration schedule prepared to examine the anticancer effect of NK cells against lung cancer according to the number of NK cells.

FIG. 8b shows the tumor volume inhibitory effect of NK cells against lung cancer according to the number of NK cells.

V.C: vehicle control group; and

Dox.hcl: Doxorubicin HCL.

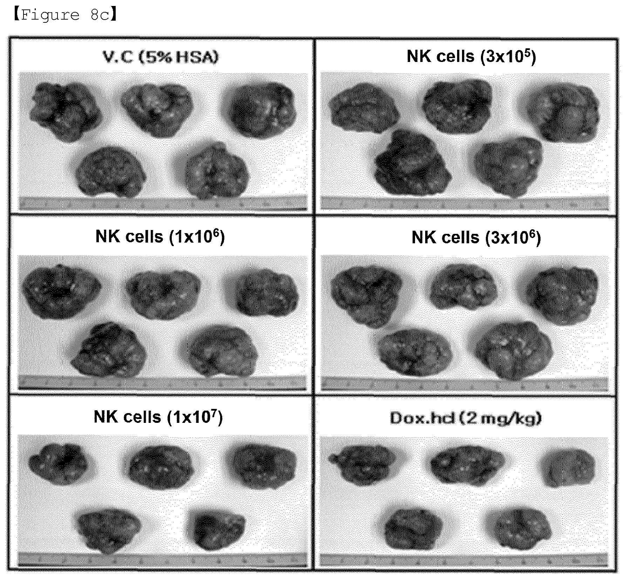

FIG. 8c shows the tumor weight-reducing effect of NK cells against lung cancer according to the number of NK cells.

V.C: vehicle control group; and

Dox.hcl: Doxorubicin HCL.

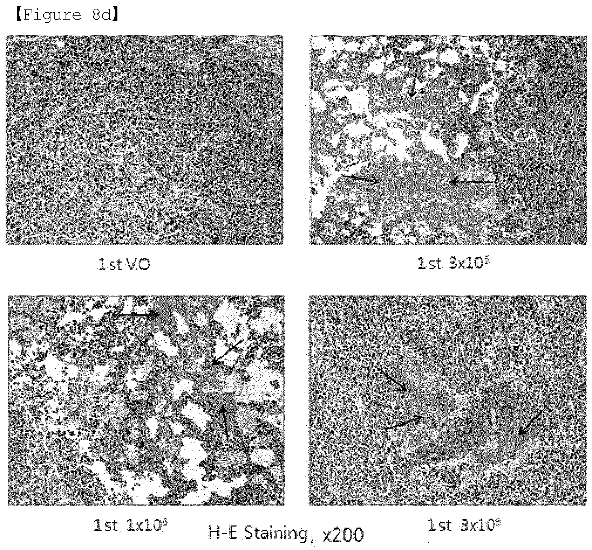

FIG. 8d shows the results of H-E staining performed to confirm that NK cells infiltrate into tumor tissue.

V.O: serum-free control group;

arrow: dead cancer cells; and

CA: cancer cells.

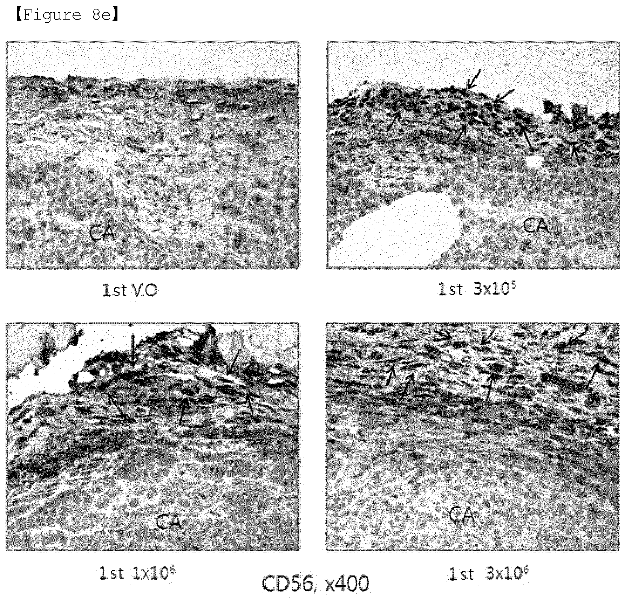

FIG. 8e shows the results of CD56 analysis performed to confirm that NK cells infiltrate into tumor tissue.

V.O: serum-free medium control group,

arrow: CD56-positive cells, and

CA: cancer cells.

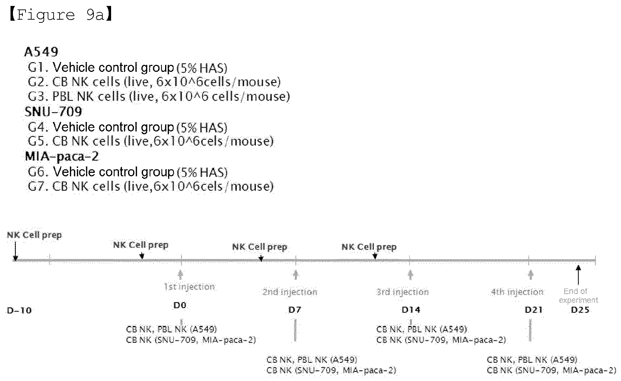

FIG. 9a shows an administration schedule prepared to examine the anticancer effects of NK cells against lung cancer, liver cancer and pancreatic cancer.

CB NK cells: umbilical cord blood-derived NK cells; and

PBL NK cells: peripheral cell-derived NK cells.

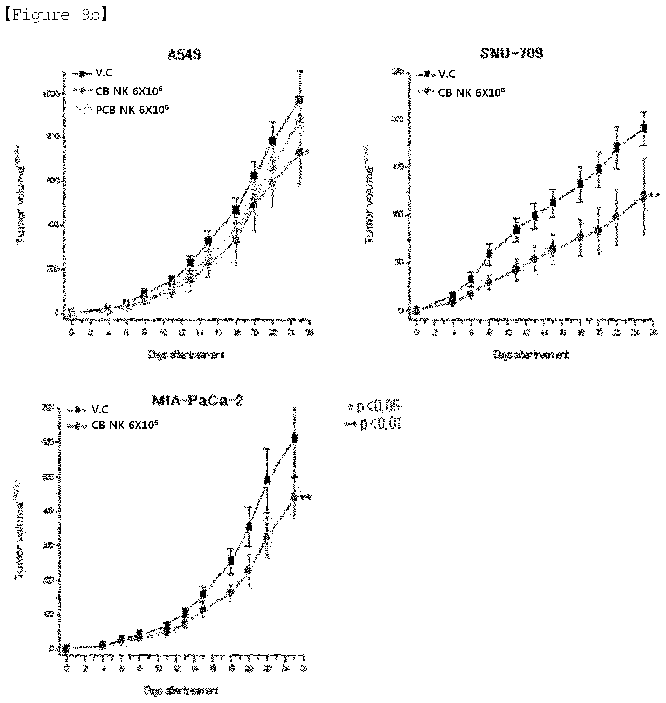

FIG. 9b shows the tumor volume inhibitory effects of NK cells against lung cancer (A549), liver cancer (SNU-709) and pancreatic cancer (MIA-PaCa-2).

V.C: vehicle control group,

CB NK cells: umbilical cord blood-derived NK cells; and

PBL NK cells: peripheral cell-derived NK cells.

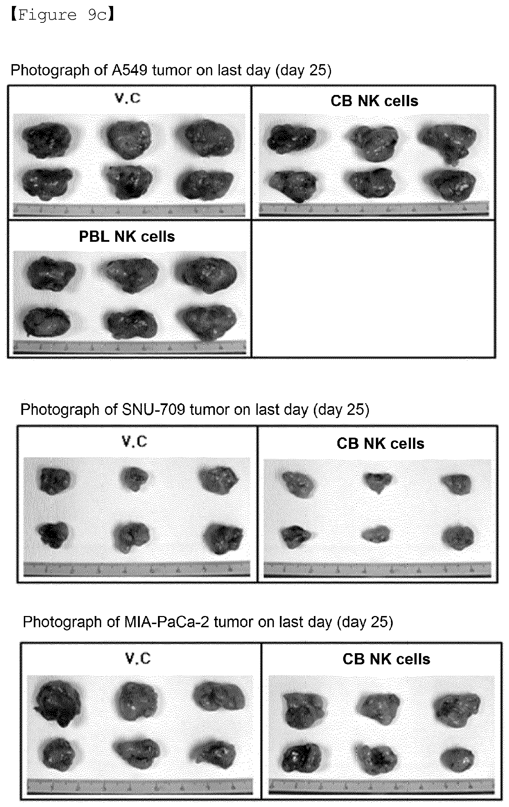

FIG. 9c shows the tumor weight-reducing effects of NK cells against lung cancer (A549), liver cancer (SNU-709) and pancreatic cancer (MIA-PaCa-2).

V.C: vehicle control group,

CB NK cells: umbilical cord blood-derived NK cells; and

PBL NK cells: peripheral cell-derived NK cells.


Best Mode

Hereinafter, the present invention describes in detail with reference to examples and experimental examples.


Example 1: Production of NK Cells

Umbilical cord blood and peripheral blood, provided for research purposes from the Department of Obstetrics and Gynecology, Konyang University Hospital (Korea) and the Department of Obstetrics and Gynecology, Chungnam National University Hospital (Korea) (approved by the IRB of each hospital), were diluted at 2:1 with RPMI 1640 to prepare a blood dilution. The prepared blood dilution was placed carefully in the upper layer of Ficoll-Paque, and then centrifuged at 2,000 rpm for 30 minutes to obtain a mononuclear cell layer (MNC layer). Cells were carefully collected from the mononuclear cell layer, and erythrocytes were removed from the collected cells to obtain monocytes. CD3 microbeads (Miltenyi Biotech) were added to the obtained monocytes to label with CD3, and then CD3-positive cells were removed by using CS column and Vario MACS to obtain CD3-negative cells. Specifically, the CD3 microbeads (Miltenyi Biotech) recognized CD3 ε chains and capture CD3-positive cells from the monocytes so as to be magnetized. Then, among the monocytes, CD3-positive cells to which the microbeads were attached were passed through a MACS column reacting with a magnet, and thus CD3-positive cells remained in the column, and only CD3-negative cells were separated from the column.

Blood was diluted with saline and treated with a suitable amount of RosetteSep™ capable of cross-linking to CD3-positive cells, depending on the counted number of cells, after which the blood was agitated at room temperature for 20 minutes. After agitation, the blood were diluted 2-fold and placed onto Ficoll-Paque solution in such a manner that layers would not be mixed, after which the resulting solution was centrifuged at 2,000 rpm at room temperature for 20-30 minutes. After removal of the supernatant, the separated monocyte layer was collected and washed to obtain CD3-negative cells. The RosetteSep component is a tetrameric complex comprising mouse- and rat-derived monoclonal antibodies, glycoporin A antibody, and P9 antibody or P9 F(ab') antibody serving as a support. In the process of isolating the CD3-negative cells, the tetrameric complex of RosetteSep added to the blood crosslinks to CD3-positive cells in the blood to form immunorosettes, and the immunorosettes having a density higher than that of Ficoll is located below Ficoll by Ficoll-based density gradient centrifugation, and CD3-negative cells that did not bind to the tetrameric complex are located above Ficoll and isolated.

The isolated CD3-negative cells were seeded into a T75 flask at a concentration of $1 \times 10^6$ cells/ml, and cultured with IL-15 and IL-21 in alpha-MEM complete medium under the conditions of 37°C and 5% $CO_2$ for 10-21 days. During culture, the concentration of the cells did not exceed $2 \times 10^6$ cells/ml, and was adjusted to a concentration of $1 \times 10^6$ cells/ml by use of a medium having the same conditions as those of the original medium. On 4, 8, 14, 18 and 21 days, the cell number was counted, and on 4, 8, 14 and 21, the cells were stained with CD3 and CD56 antibodies, and the proportion of CD3⁻CD56⁺ NK cells was analyzed by FACS according to a known method.

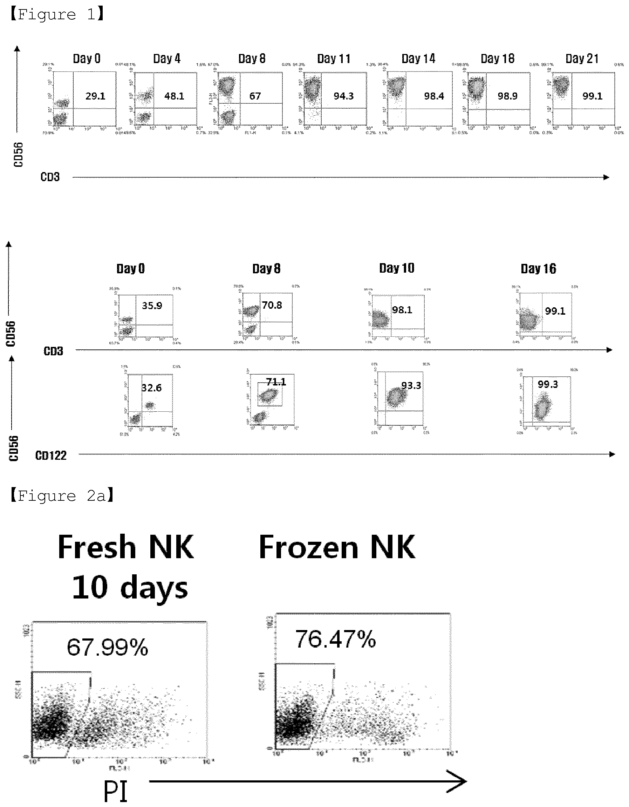

As a result, as shown in FIG. 1, differentiation of NK cells from the CD3-negative cells isolated from umbilical cord blood (the top of FIG. 1) and peripheral blood (the bottom of FIG. 1) was induced after 11-21 days of culture.

Example 2: Production of Cryopreserved NK Cells

2-1: Production of Cryopreserved NK Cells from Differentiated NK Cells

The NK cells (after 10 days of culture) produced by the method of Example 1 was cryopreserved to produced cryopreserved NK cells. The frozen storage was performed using a cryopreservation medium (Cryostor) containing 10% DMSO (dimethyl sulfoxide) under serum-free, protein-free and animal component-free conditions, and the differentiated NK cells were frozen at a concentration of 2.25 x $10^7$ cells/1.5 ml (1.5 x $10^7$ cells/ml). The freezing was performed using a cryopreservation box containing isopropyl alcohol, and the cells were cooled stepwise from -70°C (deep freezer) and finally preserved at - 200°C (LN2).

The cryopreservation was performed for 1 month. Immediately before use, the cryopreserved cells were thawed rapidly at 37°C by washing the cells with saline to remove the cryopreservation medium. The thawed cryopreserved NK cells were analyzed by FACS to determine the proportion of CD3⁻CD56⁺ NK cells, the proportion of NK receptors, the cell viability, and their cytotoxicity to CML (chronic myelogenous leukemia) cells, and the characteristics thereof were compared with fresh NK cells (fresh NK cells, not cryopreserved) of the same origin.

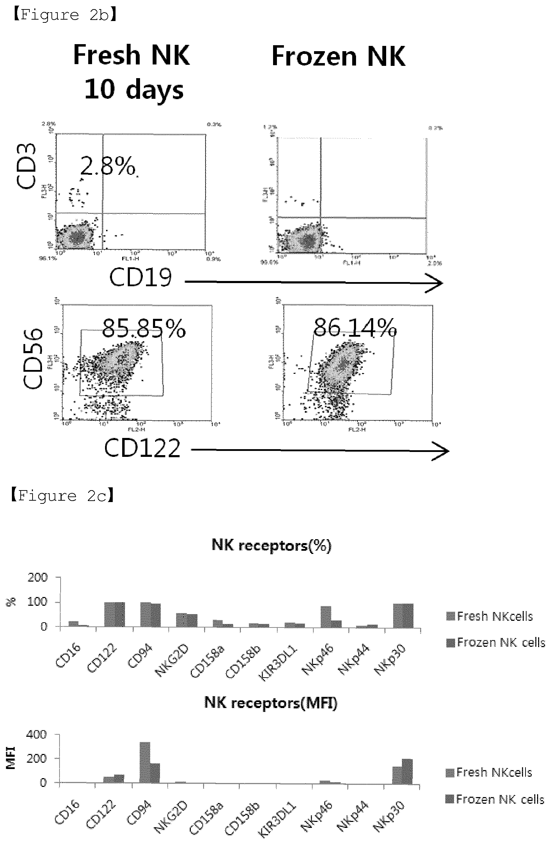

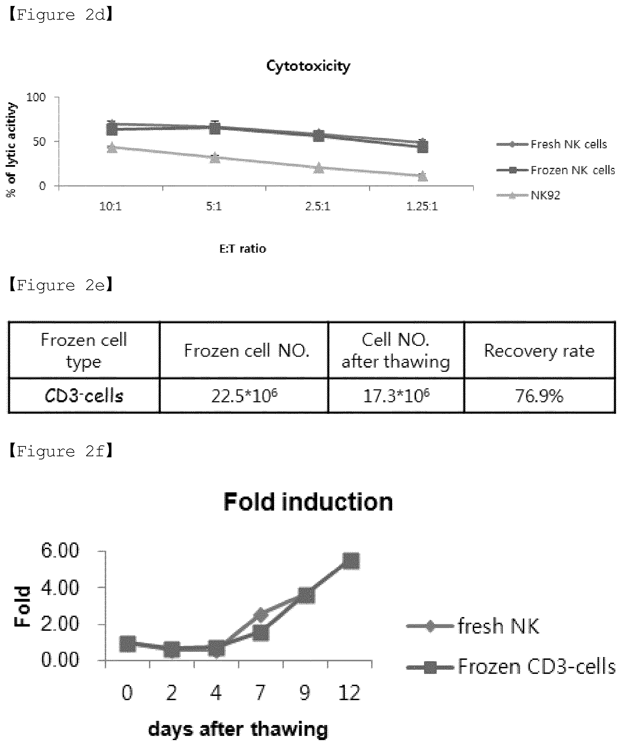

The results are shown in FIG. 2a (1. viability), FIG. 2b (2. degree of differentiation), FIG. 2c (3. receptors), FIG. 2d (4. cytotoxicity). As can be seen in FIGS. 2a to 2d, it was shown that the thawed cryopreserved NK cells had characteristics similar to those of fresh NK cells.

2-2: Production of NK Cells from Cryopreserved CD3-Negative Cells

According to the same method as described in Example 1, CD3-negative cells were obtained from umbilical cord blood, and the CD3-negative cells were cryopreserved using the same cryopreservation medium as described in Example 2-1. The concentration of the CD3-negative cells during cryopreservation was 2.25 x $10^7$ cells/1.5 ml (1.5 x $10^7$ cells/ml), and the cryopreservation was performed for about 1 month.

To obtain differentiated NK cells from the cryopreserved CD3-negative cells, the frozen cells were thawed and cultured in the same manner as described in Example 1. On 0, 2, 4, 7, 9 and 12 days after thawing, the number and viability of the cells were measured, and on 0, 7 and 9 days after thawing, the proportion of CD3-CD56+ NK cells was analyzed by FACS.

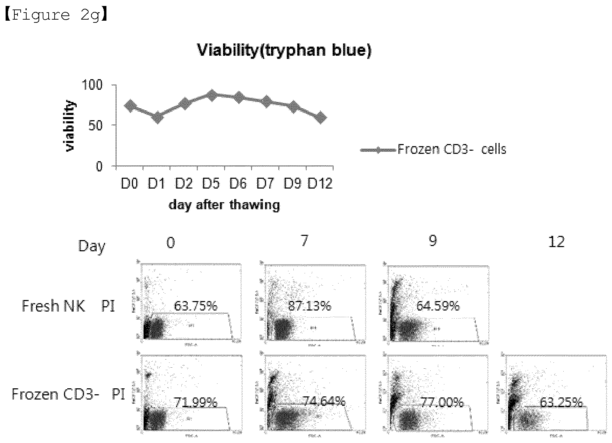

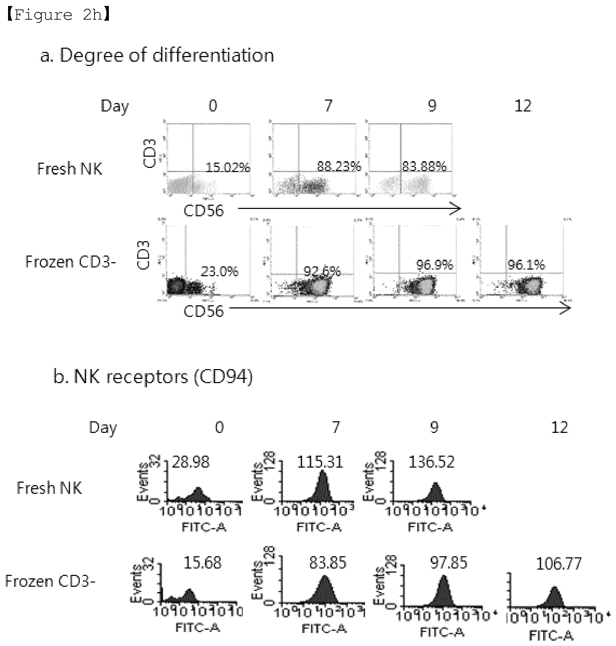

As a result, it was shown that the NK cells obtained from the cryopreserved CD3-negative cells showed characteristics similar to those of fresh NK cells with respect to all the recovery rate of cells recovered after thawing (FIG. 2e), the number of cells (FIG. 2f), the viability of cells (FIG. 2g) and the degree of differentiation (FIG. 2h).

Reference example: Examination of In Vivo Distribution of NK Cells in Mice

In order to examine the tissue distribution of the fresh NK cells produced in Example 1, the following experiment was performed.

To examine the distribution of NK cells, the NK cells were labeled with DiR. Specifically, $1 \times 10^7$ cells were suspended in 10 ml of $1 \times PBS$ (phosphate buffered saline) containing 3.5 $\mu$g/ml of DiR (1,1'-dioctadecyl-3,3,3',3'-tetramethylindotricarbocyanine, Sigma, USA) dye and 0.5% ethanol, and were incubated at 37°C for 1 hour. After incubation, the cells were washed twice with $1 \times PBS$ and stained with tryphan blue to examine the *in vivo* distribution of the NK cells.

In order to examine the tissue distribution of the DiR-labeled NK cells at varying time points, 5% HSA or DiR-labeled NK cells were injected intravenously into BALB/C female nude mice at cell concentrations of $1 \times 10^3$, $1 \times 10^4$, $5 \times 10^4$, $1 \times 10^5$, $5 \times 10^5$, $1 \times 10^6$ and $5 \times 10^6$ cells, and after a given amount of time, and the distribution of the NK cells in the mouse or the major organs (liver, spleen, heart and kidney) extracted from the mice by autopsy was examined using a live animal imaging system (PHOTONE IMAGER, Biospace) according to the manufacturer's protocol.

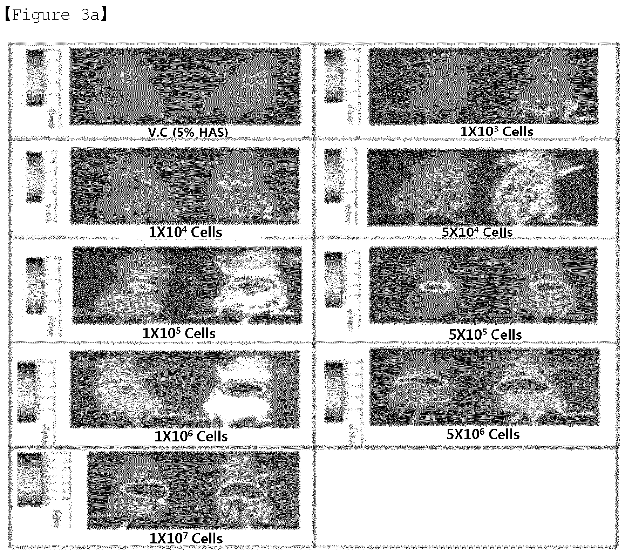

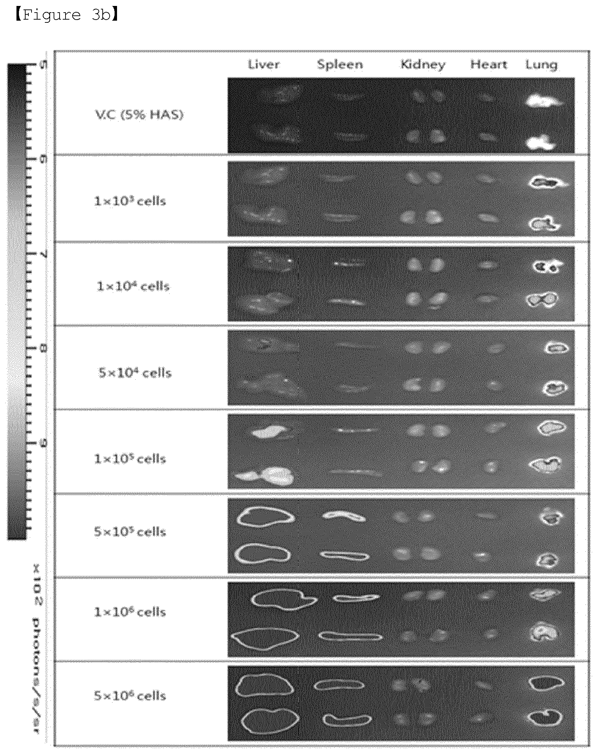

To measure the detection limit of the DiR-labeled NK cells at varying concentrations, the *in vivo* distribution of the DiR-labeled NK cells was examined 24 hours after injection of the cells. As a result, as shown in FIG. 3a, at cell concentrations equal to or lower than $5 \times 10^4$ cells, the distribution pattern of the DiR-labeled NK cells was not clear, and at cell concentrations equal to or higher than $1 \times 10^5$ cells, the image signal was strongly detected in the abdomen of the mice in a concentration-dependent manner (FIG. 3a). Furthermore, the major intra-abdominal organs (liver, spleen, kidney, heart and lung) were extracted and imaged, and as a result, it was shown that, at cell concentrations equal to or lower than $5 \times 10^4$ cells, a weak distribution of the NK cells was observed only in the lung, but at cell concentrations equal to higher than $1 \times 10^5$ cells, the image signal was strongly detected in the liver, the spleen and the lung in a concentration-dependent manner (FIG. 3b).

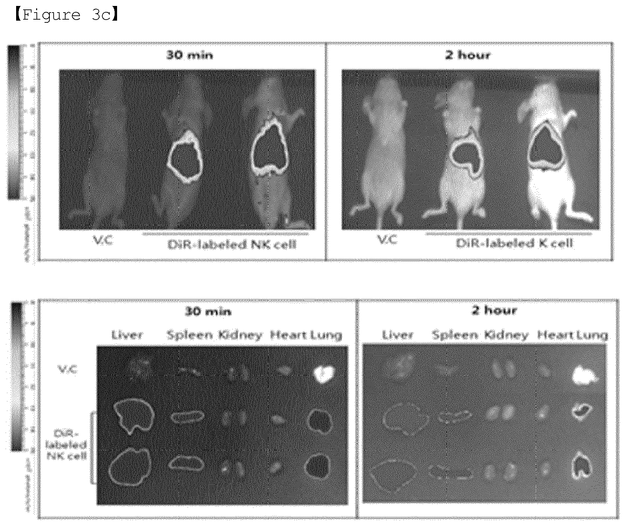

Next, in order to examine the distribution of the DiR-labeled NK cells in tissue, $1 \times 10^7$ cells were administered intravenously into the mice, and after 30 minutes and 2 hours, the distribution of the cells was measured. As a result, as shown in FIG. 3c, the distribution of the DiR-labeled NK cells in the abdomen was strongly detected from the start of the measurement, and image signals, which were 4-fold and 3.8-fold higher than the vehicle control group, were detected after 30 minutes and 2 hours, respectively. In addition, the liver, spleen, kidney, heart and lung, which are the major intra-abdominal organs, were extracted and imaged, and as a result, the distribution of the DiR-labeled NK cells in the liver, spleen and lung was strongly detected (FIG. 3c). Particularly, in the liver, image signals, which were about 9.5-fold and 5.1-fold higher than those in the vehicle control group, were detected at 30 minutes and 2 hours, respectively.

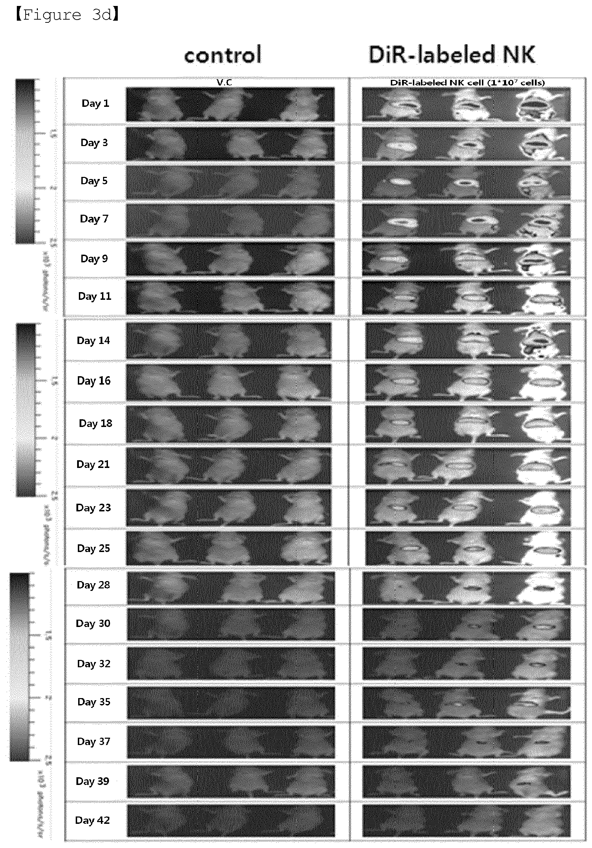

Next, the distribution was measured three times a week during a period ranging from one day after intravenous administration of the DiR-labeled NK cells to the day on which the DiR-labeled NK cells were not detected. As a result, as shown in FIG. 3d, up to 14 days after administration, the DiR-labeled NK cells were strongly detected, and after 14 days, the image signal started to be weakened, and on 42 days, the DiR-labeled NK cells were not detected in the measured image (FIG. 3d). The above results indicate that the NK cells of the present invention, after administered *in vivo,* survive *in vivo* for at least 30 days, and are abundantly distributed in lung, liver, spleen and the like.

Reference example 1: Examination of the Anticancer Effect of Administration of Fresh NK Cells against Colorectal Cancer

1-1: Examination of Tumor Volume Inhibition according to the Number of Administrations of NK Cells and the Use of NK Cells in combination with IL-2

In order to examine the anticancer effect of the NK cells produced by the method of Example 1, mouse models xenografted with human colorectal cancer SW620 cells (Korea Research Institute of Bioscience and Biotechnology, Korea) were used.

Specifically, SW620 cells were suspended in PBS at a concentration of $2 \times 10^7$ cells/ml, and then injected subcutaneously into the axilla between the right shoulder and the chest wall in an amount of 0.3 ml per mouse. At 2 hours after injection of the cancer cells, the NK cells were injected into the mice at concentrations of $3 \times 10^5$, $1 \times 10^6$, $3 \times 10^6$ and $1 \times 10^7$ cells/mouse. In addition, IL-2 was diluted in PBS, and the mice were treated with the IL-2 dilution at an IL-2 concentration of 10,000 U/mouse. During the experimental period, 0.2 ml of the NK cells were injected into the tail vein of each mouse once a week, a total of four times (0, 7, 14 and 21 days). Then, to measure the change in the tumor volume, the sizes in the three directions of the tumor were measured using vernier calipers a total of 7 times during a period ranging from the day of start of drug administration to the day of autopsy, and then the tumor cell volume was measured using the following equation 1:

$$\text{Equation 1}$$

$$\text{Tumor cell volume} = (\text{length} + \text{width} + \text{height})/2$$

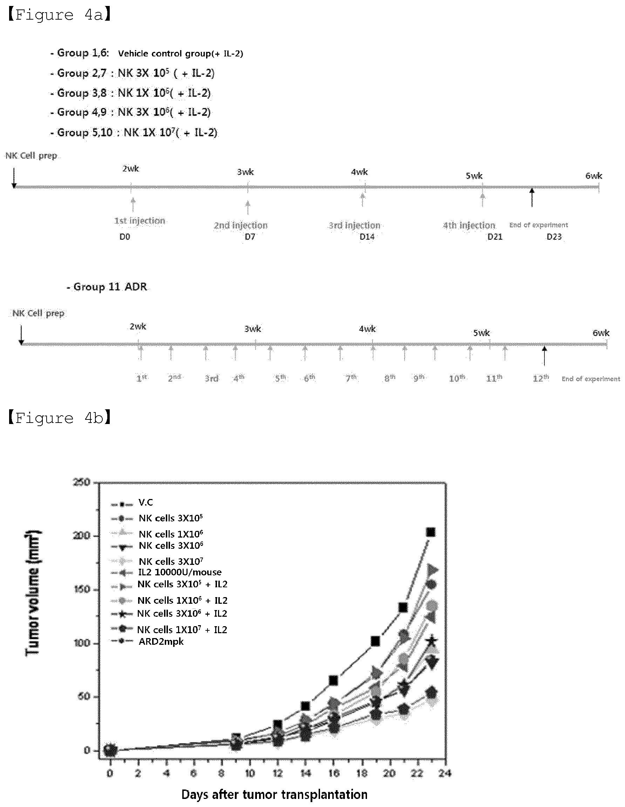

As a result, as shown in FIG. 4b, the groups injected with the NK cells at concentrations of $3 \times 10^5$, $1 \times 10^6$, $3 \times 10^6$ and $1 \times 10^7$ cells/mouse showed tumor growth inhibitions of 23.8%, 53.4% ($p < 0.001$), 59.4% ($p < 0.001$) and 76.8% ($p < 0.001$), respectively, compared to that in the vehicle control group, and the positive control group administered with adriamycin showed a tumor growth inhibition of 58.3% ($p < 0.001$). In addition, when the mice were treated with the NK cells in combination with IL-2, the groups injected with the NK cells at concentrations of $3 \times 10^5$, $1 \times 10^6$, $3 \times 10^6$ and $1 \times 10^7$ cells/mouse showed tumor growth inhibitions of 17.0%, 33.8% ($p < 0.01$), 49.8% ($p < 0.01$) and 73.5% ($p < 0.001$), respectively (FIG. 4b).

1-2: Examination of Tumor Weight Reduction according to the Number of Administrations of NK cells and the Use of NK Cells in combination with IL-2

In order to examine the anticancer effects of the NK cells according to the number of administration of the NK cells and the use of the NK cells in combination with IL-2, the weights of tumors in drug-treated mice were measured.

According to the same method as described in Experimental Example 1-1, mice were treated with the NK cells alone or in combination with IL-2. On day 23, the mice were sacrificed using $CO_2$ gas, and tumors were separated from the sacrificed mice and weighed using a chemical balance. The tumors were photographed and fixed in liquid nitrogen. All the measurements were analyzed by t-TEST to determine the statistical significance between the vehicle control group and the administered groups.

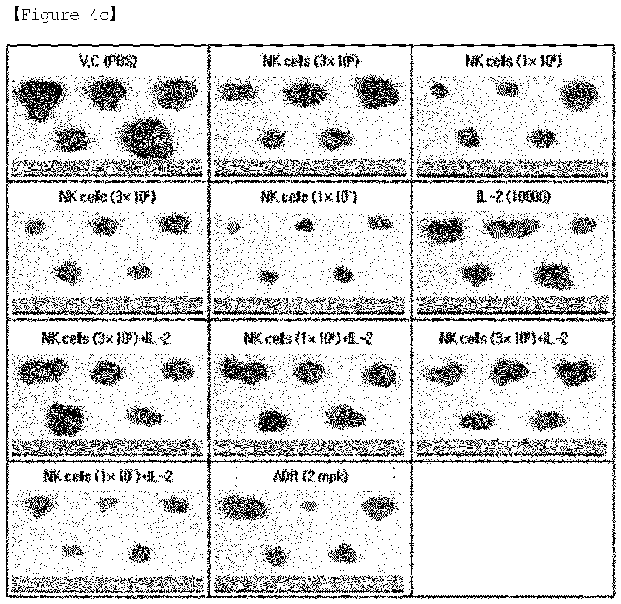

As a result, as shown in FIG. 4c, the groups injected with the NK cells at concentrations of $3 \times 10^5$, $1 \times 10^6$, $3 \times 10^6$ and $1 \times 10^7$ cells/mice showed tumor weight reductions of 23.4%, 54.0%, 59.1% ($p < 0.05$) and 78.8% ($p < 0.01$), respectively, compared to the vehicle control group (FIG. 4c). In addition, when the mice were treated with the NK cells in combination with IL-2, the groups injected with the NK cells at concentrations of $3 \times 10^5$, $1 \times 10^6$, $3 \times 10^6$ and $1 \times 10^7$ cells/mice showed tumor weight reductions of 17.0%, 34.3%, 47.0% and 75.6% ($p < 0.01$), respectively, and the positive control group administered with adriamycin showed a tumor weight reduction of 58.2% ($p < 0.05$) (FIG. 4c).

1-3: Examination of Cytotoxicity according to the Number of NK Cells and the Use of NK Cells in combination with IL-2

In order to examine the cytotoxicity of the NK cells to the mice of Experimental Example 1-1 according to administration of the NK cells alone or in combination with IL-2, the change in body weight and general symptoms of the mice were observed.

As a result, in the groups administered with the NK cells alone or in combination with IL-2, a normal increase in the body weight was observed without general symptoms during the experimental period, unlike the vehicle control group. However, the positive control group administered with adriamycin showed a statistically significant weight reduction of 21.8% ($p < 0.01$).

In conclusion, the NK cells of the present invention, when administered alone or in combination with IL-2, did not show general symptoms and toxic symptoms such as weight loss. Regarding the anticancer effects, the group administered with the NK cells alone showed an excellent tumor growth inhibition of 70% or more, and the group administered with the NK cells in combination with IL-2 did not show increased effects compared to the group administered with the NK cells alone.

Reference example 2: Examination of Anticancer Effects of NK Cells according to the Incubation, Preservation Conditions and Administration Schedules of NK Cells

2-1: Examination of Tumor Size Inhibition according to the Incubation, Preservation Conditions and Administration Schedules of NK Cells

In order to examine the anticancer effects of the NK cells of the present invention according to the NK cell incubation method (comprising removing CD3-positive T cells by Rosettesep or not comprising the removal process), preservation conditions (fresh cells or cold-preserved cells) and administration schedules (once a week for 4 weeks, or twice a week for 2 weeks).

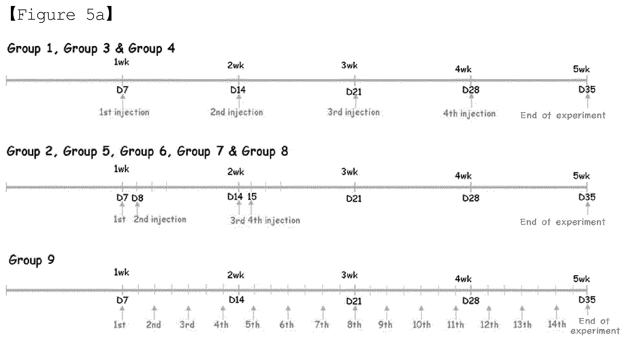

Specifically, NK cells were divided according to culture conditions into fresh NK, fresh NK (w/o Rosettesep), 4°C cold-preserved NK and 4°C cold-preserved NK (w/o Rosettesep), and the fresh NK cells were produced in the same manner as described in Example 1. The fresh NK (w/o Rosettesep) cells were produced in the same manner as described in Example 1, except that the step of removing CD3-positive cells was omitted. The 4°C cold-preserved NK cells were obtained by preserving fresh NK cells at 4°C for 12 hours, and the 4°C cold-preserved NK (w/o Rosettesep) cells were obtained by producing NK cells without the step of removing CD3-positive cells, and preserving the produced cells at 4°C for 12 hours. In order to administer the produced NK cells to mouse models, cancer cells were transplanted into mice in the same manner as described in Experimental Example 1-1, and when the average tumor volume reached about 50.0 mm$^3$, and the NK cells were administered to the mice at a concentration of $3 \times 10^6$ cells/mouse, and 0.2 ml of the NK cells were injected into the tail vein of each mouse. In the administration schedule, the NK cells were administered once a week for 4 weeks or administered twice a week for 2 weeks (FIG. 5a).

In order to examine the toxicity of the NK cells during the experimental period, the weight change and general symptoms of the mice were observed. As a result, in the groups administered with the NK cells once a week for 4 weeks [fresh NK, fresh NK (w/o Rosettesep)] and twice a week for 2 weeks [fresh NK, fresh NK (w/o Rosettesep), 4°C cold-preserved NK, or 4°C cold-preserved NK (w/o Rosettesep)], a normal increase in the weight was observed without general symptoms during the experimental period, compared to the vehicle control group. However, in the positive control group administered with adriamycin, two dead animals and a statistically significant weight reduction of 31.5% (p<0.001) were observed.

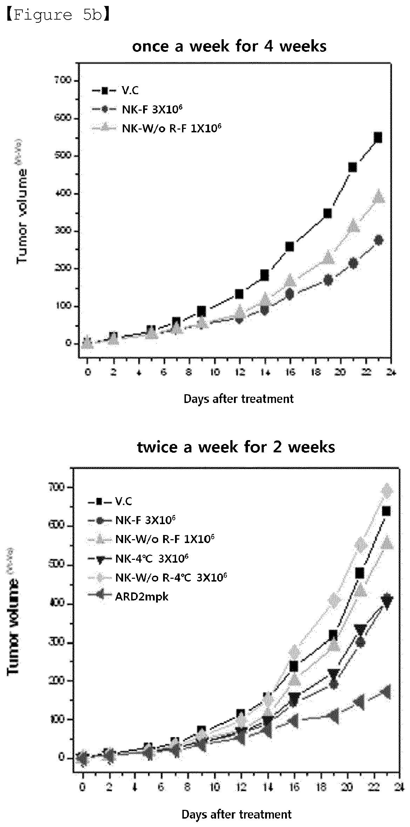

In addition, in order to examine the anticancer effects of the NK cells according to the incubation, preservation conditions and administration schedules of the NK cells, the change in tumor volume was examined. As a result, as shown in FIG. 5b, the group, administered with fresh NK and fresh NK (w/o Rosettesep) cells once a week for 4 weeks at a concentration of $3 \times 10^6$ cells/mouse, showed tumor growth inhibitions of 50.8% (p<0.001) and 32.7% (p<0.05), respectively, compared to the vehicle control group, and the groups, administered with fresh NK, fresh NK (w/o Rosettesep) and 4°C cold-preserved NK cells twice a week for 2 weeks, showed tumor growth inhibitions of 35.4%, 10.8% and 33.0%, respectively. The positive control group administered with adriamycin showed a tumor growth inhibition of 71.7% (p<0.01). However, the group administered with 4°C cold-preserved NK (w/o Rosettesep) cells showed no tumor growth inhibition. This suggests that, when the process of removing CD3-positive T cells is not performed, the proportion of NK cells is low, and thus the anticancer effect of the NK cells is reduced. Thus, it can be seen that the process of removing CD3-positive T cells is necessary to increase the anticancer effect of the NK cells.

2-2: Examination of Tumor Weight Reduction According to the Incubation, Preservation Condition and Administration Schedule of NK Cells

In order to the anticancer effect of the NK cells of the present invention according to the incubation method, preservation condition and administration schedule of the NK cells, the reduction in tumor weight was examined.

Specifically, cancer cells was transplanted into mice in the same manner as described in Experimental Example 1-1, and NK cells were administered under the same conditions as described in Experimental Example 2-1. On 27 days after drug administration, the tumor was excised and weighed.

As a result, as shown in FIG. 5c, the groups, administered with fresh NK and fresh NK (w/o Rosettesep) cells once a week for 4 weeks at a concentration of $3 \times 10^6$ cells/mouse, showed tumor weight reductions of 49.0% (p<0.001) and 33.1% (p<0.05), compared to the vehicle control group. Furthermore, the groups, administered with fresh NK, fresh NK (w/o Rosettesep) and 4°C cold-preserved NK cells twice a week for 2 weeks, showed tumor weight reductions of 30.3%, 7.2% and 29.0%, respectively, and the positive control group administered with adriamycin showed a tumor growth inhibition of 70.2% (p<0.05) (FIG. 5c).

In conclusion, when the NK cells incubated under fresh NK incubation conditions were administered once a week for 4 weeks at a concentration of $3 \times 10^6$ cells/mouse, these cells showed excellent anticancer effects. In addition, when the NK cells were administered twice a week for 2 weeks, the fresh NK and the 4°C cold-preserved NK cells showed higher anticancer activities higher than the fresh NK (w/o Rosettesep) and the 4°C cold-preserved NK (w/o Rosettesep) cells.

Reference example 3: Examination of Anticancer Effect according to Freezing or not of NK Cells

Example 3-1: Examination of Tumor Volume Inhibition according to Freezing or not of NK Cells

In order to examine the anticancer effect of the NK cells of the present invention according to freezing or not of the NK cells, the reduction in tumor volume was examined.

Specifically, cancer cells were transplanted into mice in the same manner as described in Experimental Example 1-1, and after 2 hours, the NK cells were administered to the mice at a concentration of $3 \times 10^6$ cells/mouse, and 0.2 ml of the NK cells were injected into the tail vein of each mouse. Herein, the NK cells were administered under the following conditions: fresh NK cells (once a week for 4 weeks, a total of four times), thawed cryopreserved NK cells (once a week for 4 weeks, a total of four times), thawed cryopreserved NK cells (twice a week for 4 weeks, a total of eight times), and fresh NK cells (once a week for one week) plus thawed cryopreserved NK cells (once a week for three weeks, a total of three times). Vehicle control groups were administered with 5% HAS and serum free medium, and a positive control group was administered intraperitoneally with 1 mg/kg of adriamycin at 2-day intervals (FIG. 6a).

To examine toxicity during the experimental period, the weight change and general symptoms of the mice were observed. As a result, in all the groups administered with the NK cells, a normal increase in the weight was observed without general symptoms during the experimental period, compared to the vehicle control groups. However, in the positive control group administered with adriamycin, two dead animals and a statistically significant weight reduction of 32.1% ($p < 0.001$) appeared.

In addition, in order to examine the anticancer effect of the NK cells according to freezing or not of the NK cells, the change in tumor volume on day 27 was examined. As a result, as shown in FIG. 6b, the groups, administered with fresh NK cells (a total of four times), thawed cryopreserved cells (a total of eight times), and fresh NK cells (once) plus thawed cryopreserved NK cells (a total of three times), showed tumor growth inhibitions of 58.8% ($p < 0.001$), 45.2% ($p < 0.001$) and 19.2%, respectively, and the positive control group showed a tumor growth inhibition of 60.1% ($p < 0.01$) (FIG. 6b).

3-2: Examination of Tumor Weight Reduction according to Freezing or not of NK Cells

In order to examine the anticancer effect according to freezing or not of the NK cells of the present invention, the reduction in tumor weight was examined.

Specifically, cancer cells were transplanted into mice in the same manner as described in Experimental Example 1-1, and then NK cells were administered to the mice under the same conditions as described in Experimental Example 4-1. On 27 days after drug administration, the tumor was excised and weighed.

As a result, as shown in FIG. 6c, the groups, administered with fresh NK cells (a total of 4 times), thawed cryopreserved NK cells (a total of 8 times), and fresh NK cells (once) plus thawed cryopreserved NK cells (a total of 3 times), showed tumor weight reductions of 58.5% ($p < 0.001$), 46.2% ($p < 0.01$) and 19.5%, respectively, and the positive control group showed a tumor weight reduction of 60.5% ($p < 0.05$) (FIG. 6c).

In conclusion, the above-described results indicate that, when the fresh NK cells (once a week for 4 weeks, a total of 4 times) or the thawed cryopreserved NK cells (twice a week for 4 weeks, a total of 8 times) are administered at a concentration of $3 \times 10^6$ cells/mouse, they show significant anticancer effects without general symptoms and toxic symptoms such as weight loss.

Reference example 4: Examination of Anticancer Effects of NK Cells according to Freezing or not of NK Cells and the Number of Administrations of NK Cells

4-1: Examination of Tumor Volume Inhibition according to Freezing or not of NK Cells and the Number of Administrations of NK Cells

In order to examine the anticancer effects of the NK cells of the present invention according to freezing or not of the NK cells and the number of administrations of the NK cells, the reduction in tumor volume was examined.

Specifically, cancer cells were transplanted into mice in the same manner as described in Experimental Example 1-1. After 2 hours, fresh NK cells and thawed cryopreserved NK cells were administered to the mice at concentrations of 3 x $10^6$ cells/mouse and 6 x $10^6$ cells/mouse, and 0.2 ml of the NK cells were injected into the tail vein of each mouse. Herein, the NK cells were administered under the following conditions: 3 x $10^6$ fresh NK cells/mouse (once a week for 4 weeks, a total of 4 times) ; 6 x $10^6$ fresh NK cells/mouse (once in two weeks for 4 weeks; a total of two times); and fresh NK cells ($3 \times 10^6$ cells/mouse; once a week for 1 week) plus thawed cryopreserved cells ($3 \times 10^6$ cells/mouse; twice a week for 3 weeks, a total of 6 times); thawed cryopreserved NK cells (serum free medium; twice a week for 4 weeks, a total of 8 times); and thawed cryopreserved NK cells (distilled water; twice a week for 4 weeks, a total of 8 times). Vehicle control groups were injected intravenously with the same amount of 5% HAS, serum-free medium and distilled water, respectively, and a positive control group was administered intraperitoneally with 2 mg/kg of doxorubicin HCL at 2-day intervals (FIG. 7a).

In order to examine toxicity during the experimental period, the weight change and general symptoms of the animals were observed. As a result, in all the groups injected with the NK cells, a normal increase in the weight was observed without general symptoms, compared to the vehicle control group. However, in the positive control group administered with doxorubicin HCL, two dead animals during the administration period and a statistically significant weight reduction of 25.0% (p<0.01) on the last day appeared.

In addition, in order to examine the anticancer effects of the NK cells according to freezing or not of the NK cells and the number of administrations of the NK cells, the change in tumor volume on day 26 was examined. As a result, as shown in FIG. 7b, the groups, administered with 3 x $10^6$ fresh NK cells/mouse (a total of 4 times), 6 x $10^6$ fresh NK cells/mouse (a total of two times), fresh NK cells ($3\times10^6$ cells/mouse; once) plus thawed cryopreserved NK cells ($3\times10^6$ cells/mouse; a total of 6 times), thawed cryopreserved NK cells (serum free medium; a total of 8 times), and thawed cryopreserved NK cells (distilled water; a total of 8 times), showed tumor growth inhibitions of 68.3% (p<0.001), 61.5% (p<0.001), 63.7% (p<0.001), 55.1% (p<0.01) and 38.8%, respectively. The positive control group administered with doxorubicin HCl showed a tumor growth inhibition of 72.9% (p<0.01) on the last day (FIG. 7b).

4-2: Examination of Tumor Weight Reduction according to Freezing or not of Cells, Number of Cells and the Number of Administrations of Cells

In order to examine the anticancer effects of the NK cells of the present invention according to freezing or not of the NK cells, the number of the NK cells and the number of administrations of the NK cells, the reduction in tumor weight was examined.

Specifically, cancer cells were transplanted into mice in the same manner as described in Experimental Example 1-1, and then NK cells were administered to the mice under the same conditions as described in Experimental Example 4-1. On 26 days after drug administration, the tumor was excised and weighed. As a result, as shown in FIG. 7c, the groups, administered with 3 x $10^6$ fresh NK cells/mouse (a total of 4 times), 6 x $10^6$ fresh NK cells/mouse (a total of two times), fresh NK cells ($3\times10^6$ cells/mouse; once) plus thawed cryopreserved NK cells ($3\times10^6$ cells/mouse; a total of 6 times), thawed cryopreserved NK cells (serum free medium; a total of 8 times), and thawed cryopreserved NK cells (distilled water; a total of 8 times), showed tumor weight reductions of 68.6% (p<0.001), 61.8% (p<0.01), 59.1% (p<0.01), 50.2% (p<0.05) and 40.8% (p<0.05), respectively. The group administered with the positive control Doxorubicin HCl showed a tumor weight reduction of 70.4% (p<0.01) (FIG. 7c).

In conclusion, the above-described results indicated that, when fresh NK cells (once a week for 4 weeks, a total of 4 times; or once in two weeks for 4 weeks, a total of two times), thawed cryopreserved NK cells (serum free medium; twice a week for 4 weeks, a total of 8 times), and fresh NK cells (once a week for 1 week) plus thawed cryopreserved NK cells (twice a week for 3 weeks, a total of 6 times), were injected into the tail veins of each mouse at the above-described concentrations, these cells showed statistically significant excellent effects on the inhibition of tumor growth without causing general symptoms and toxic symptoms such as weight loss.

Experimental Example 5: Examination of the Anticancer Effect of NK Cells against Lung Cancer according to the Number of NK Cells

5-1: Examination of the Effects of NK Cells on Tumor Growth Inhibition and Tumor Weight Reduction according to Number of NK Cells

Using mouse models xenografted with human lung cancer NCI-H460 cells (Korea Research Institute of Bioscience and Biotechnology, Korea), the anticancer effect of the NK cells of the present invention was examined according to the number of the NK cells by intravenously injecting varying doses of the NK cells in a repeated manner.

Specifically, cancer cells were transplanted into mice in the same manner as described in Experimental Example 1-1, and when the average tumor volume reached 50.0 mm$^3$, NK cells were injected into the mice at concentrations of $3\times10^5$, $1\times10^6$, $3\times10^6$ and $1\times10^7$ cells/mouse. 0.2 ml of the NK cells were injected into the tail vein of each mouse once a week for 4 weeks (a total of 4 times). A solvent control group was administered with 5% HSA, and a positive control group was administered intraperitoneally with 2 mg/kg of doxorubicin HCL at 2-day intervals (FIG. 8a).

In order to examine toxicity during the experimental period, the weight change and general symptoms of the mice were observed. As a result, in all the groups administered with varying doses of the NK cells, a normal increase in the weight was observed without general symptoms during the experimental period, compared to the vehicle control group. However, in the positive control group administered with doxorubicin HCL, a statistically significant weight reduction of 43.3% (p<0.001) appeared.

In order to examine the anticancer effect of the NK cells, the tumor volume was measured a total of 11 times according to the method of Experimental Example 1 during a period ranging from the day of start to the day of autopsy. On the last day (day 26), the mice were sacrificed, and the tumor was separated from the sacrificed mice, and then the volume of the tumor was measured to determine the tumor growth inhibitory effect of the NK cells. As a result, as shown in FIG. 8b, the group, administered with NK cells at a concentration of $1\times10^7$ cells/mouse, showed a significant tumor growth inhibition of 47.9% (p<0.001), compared to the vehicle control group. The groups, administered with NK cells at concentrations of $3\times10^5$, $1\times10^6$ and $3\times10^6$ cells/mouse, showed tumor growth inhibition up to 10 days after administration of the NK cells, but after 10 days, the tumor growth in these group showed a tendency to increase as the inhibition rate decreased. Furthermore, the positive control group showed a tumor growth inhibition of 53.8% (p<0.001) (FIG. 8b).

In addition, the effect of the NK cells on tumor weight reduction was examined. As a result, as shown in FIG. 8c, the group, administered with NK cells at a concentration of $1\times10^7$ cells/mouse, showed a significant tumor weight reduction of 46.5% (p<0.001), compared to the vehicle control group, and the positive control group showed a tumor weight reduction of 52.4% (p<0.001) (FIG. 8c).

5-2: Examination of NK Cell Infiltration into Tumor Tissue

In order to examine the amount of NK cells that infiltrated into tumor tissue when the NK cells were administered under the conditions described in Experimental Example 5-1, the following experiment was performed.

Specifically, mouse cancer tissue was fixed in 10% formalin solution at 4°C for 12 hours. The fixed tissue was sectioned thinly and placed in PBS solution. For immunohistochemical staining of the NK cells, the tissue section was placed in 3% hydrogen peroxide solution for 30 minutes, and then placed in a solution containing 0.1 M PBS (pH 7.4), 0.1% triton X-100, serum bovine albumin and CD56 antibody (1:500, PharMigen, USA), followed by incubation at 4°C for 12 hours. Thereafter, the section was incubated in a solution containing fluorescence-labeled anti-mouse IgG (1:200, PharMigen, USA) at room temperature for 1 hour, and then observed with a microscope. Alternatively, the fixed tissue was directly stained with hematoxylin and eosin and was observed with a microscope.

As a result, as shown in FIGS. 8d and 8e, when the NK cells were administered to the mice at concentrations of $3\times10^5$, $1\times10^6$ and $3\times10^6$ cells/mouse, the number of dead cancer cells (arrow) increased in a manner dependent on the number of NK cells injected (FIG. 8d), and the number of the NK cell marker CD56-positive cells (arrow) significantly increased compared to that in the normal control group administered with the vehicle, and also CD56-positive cells mainly infiltrated around cancer tissue (FIG. 8e).

In conclusion, it was shown that, when the NK cells were injected into the tail veins of the mice once a week for 4 weeks at a concentration of $1\times10^7$ cells/mouse, these NK cells showed significant tumor inhibitory effects against human lung cancer without causing general symptoms and toxic symptoms such as weight loss.

Reference example 6: Examination of Anticancer Effects of NK Cells against Lung Cancer, Liver Cancer and Pancreatic Cancer

In order to the anticancer effects of the NK cells of the present invention against various kinds of cancer, the anticancer effects of the NK cells were examined by repeated intravenous injections into mouse models xenografted with human lung cancer A549 cells (Korea Research Institute of Bioscience and Biotechnology, Korea), liver cancer SNU-709 cells (Korea Research Institute of Bioscience and Biotechnology, Korea) and pancreatic cancer MIA-Paca-2 cells (Korea Research Institute of Bioscience and Biotechnology, Korea).

Specifically, cancer cells were transplanted into mice in the same manner as described in Experimental Example 1-1, and when the average tumor volume reached 50.0 mm$^3$, NK cells were injected into the mice at a concentration of 6 x 10$^6$ cells/mouse. 0.2 ml of the NK cells were injected into the tail vein of each mouse once a week for 4 weeks (a total of 4 times). A solvent control group was administered with 5% HSA (FIG. 9a).

In order to examine toxicity during the experimental period, the weight change and general symptoms of the mice were observed. As a result, in all the groups administered with varying doses of the NK cells, a normal increase in the weight was observed without general symptoms during the experimental period, compared to the vehicle control group.

In order to examine the anticancer effect of the NK cells, the tumor volume was measured a total of 11 times according to the method of Experimental Example 1 during a period ranging from the day of start to the day of autopsy. On the last day (day 25), the mice were sacrificed, and the tumor was separated from the sacrificed mice, and then the volume of the tumor was measured to determine the tumor growth inhibitory effect of the NK cells. As a result, as shown in FIG. 9b, in the lung cancer mouse models, the groups administered with umbilical cord blood-derived NK cells and peripheral blood-derived NK cells showed tumor growth inhibitions of 24.7% (p<0.05) and 9.0%, respectively, compared to the vehicle control group. In the liver cancer mouse models, the group administered with umbilical cord blood-derived NK cells showed a significant tumor growth inhibition of 37.7% (p<0.01). In the pancreatic cancer mouse models, the group administered with umbilical cord blood-derived NK cells showed a significant tumor growth inhibition of 28.2% (p<0.01) (FIG. 9b).

In addition, on 25 days after administration of the NK cells, the effects of the NK cells on tumor weight reduction were examined. As a result, as shown in FIG. 9c, in the lung cancer mouse models, the groups administered with umbilical cord blood-derived NK cells and peripheral blood-derived NK cells showed tumor growth inhibitions of 20.4% (p<0.01) and 10.8%, respectively, compared to the vehicle control group. In the liver cancer mouse models, the group administered with umbilical cord blood-derived NK cells showed a tumor weight reduction of 37.6% (p<0.01), and in the pancreatic cancer mouse models, the group administered with umbilical cord blood-derived NK cells showed a tumor weight reduction of 23.9% (p<0.01) (FIG. 9c).

In conclusion, it was shown that, when the NK cells of the present invention were injected into the tail veins of the mice once a week for 4 weeks at a concentration of $6 \times 10^6$ cells/mouse, these NK cells showed significant tumor inhibitory effects against human lung cancer, liver cancer and pancreatic cells without causing general symptoms and toxic symptoms such as weight loss.

## Claims

1. A method for producing cryopreserved NK cells, the method comprising the steps of:

   1) removing CD3-positive T cells from monocytes to obtain CD3-negative cells;
   2) culturing the CD3-negative cells by treating the CD3-negative cells of step 1) with IL-15 and IL-21; and
   3) freezing the cultured CD3-negative cells of step 2) in a cryopreservation medium containing 10% DMSO (dimethyl sulfoxide) under serum-free, protein-free and animal component-free conditions for 2 months or less, wherein the freezing is performed by stepwise cooling from -70°C to -200°C.

2. The method according to claim 1, wherein the method further comprises a step 4) of quick thawing the cryopreserved NK cells at 37°C, and washing out the cryopreservation medium.

3. A method of producing NK cells from frozen CD3-negative cells, the method comprising the steps of:

   1) removing CD3-positive T cells from monocytes to obtain CD3-negative cells;
   2) freezing the obtained CD3-negative cells of step 1) in a cryopreservation medium containing 10% DMSO (dimethyl sulfoxide) under serum-free, protein-free and animal component-free conditions for 2 months or less, wherein the freezing is performed by stepwise cooling from -70°C to -200°C;
   3) thawing the frozen CD3-negative cells of step 2); and
   4) culturing the thawed CD3-negative cells by treating the thawed CD3-negative cells of step 3) with IL-15 and IL-21.

## Patentansprüche

1. Verfahren zum Herstellen von kryokonservierten NK-Zellen, wobei das Verfahren die folgenden Schritte umfasst:

   1) Entfernen von CD3-positiven T-Zellen aus Monozyten, um CD3-negative Zellen zu erhalten;
   2) Kultivieren der CD3-negativen Zellen durch Behandeln der CD3-negativen Zellen aus Schritt 1) mit IL-15 und IL-21; und
   3) Einfrieren der kultivierten CD3-negativen Zellen aus Schritt 2) in einem Kryokonservierungsmedium, das 10 % DMSO (Dimethylsulfoxid) enthält, unter serumfreien, proteinfreien und tierbestandteilfreien Bedingungen über 2 Monate oder weniger, wobei das Einfrieren durch schrittweises Abkühlen von -70 °C auf -200 °C durchgeführt wird.

2. Verfahren nach Anspruch 1, wobei das Verfahren ferner einen folgenden Schritt 4) umfasst:
   schnelles Auftauen der kryokonservierten NK-Zellen bei 37 °C und Herauswaschen des Kryokonservierungsmediums.

3. Verfahren zum Herstellen von NK-Zellen aus tiefgekühlten CD3-negativen Zellen, wobei das Verfahren die folgenden Schritte umfasst:

   1) Entfernen von CD3-positiven T-Zellen aus Monocyten, um CD3-negative Zellen zu erhalten;
   2) Einfrieren der erhaltenen CD3-negativen Zellen aus Schritt 1) in einem Kryokonservierungsmedium, das 10 % DMSO (Dimethylsulfoxide) enthält, unter serumfreien, proteinfreien und tierbestandteilfreien Bedingungen über 2 Monate oder weniger, wobei das Einfrieren durch schrittweises Abkühlen von -70 °C auf -200 °C durchgeführt wird;
   3) Auftauen der eingefrorene CD3-negativen Zellen aus Schritt 2); und
   4) Kultivieren der aufgetauten CD3-negativen Zellen durch Behandeln der CD3-negativen Zellen aus Schritt 3) mit IL-15 und IL-21.

**Revendications**

1. Procédé de production de cellules NK cryopréservées, le procédé comprenant les étapes de :

   1) élimination de lymphocytes T positifs pour CD3 à partir de monocytes afin d'obtenir des cellules négatives pour CD3 ;
   2) mise en culture des cellules négatives pour CD3 par traitement des cellules négatives pour CD3 de l'étape 1) avec IL-15 et IL-21 ; et
   3) congélation des cellules négatives pour CD3 cultivées de l'étape 2) dans un milieu de cryoconservation contenant du DMSO à 10 % (diméthylsulfoxyde) dans des conditions exemptes de sérum, exemptes de protéines et exemptes de composants animaux pendant 2 mois ou moins, dans lequel la congélation est effectuée par refroidissement graduel allant de -70 °C à -200 °C.

2. Procédé selon la revendication 1, dans lequel le procédé comprend en outre une étape 4) de décongélation rapide des cellules NK cryopréservées à 37° C, et de lavage du milieu de cryoconservation.

3. Procédé de production de cellules NK à partir de cellules négatives pour CD3 congelées, le procédé comprenant les étapes de :

   1) élimination de lymphocytes T positifs pour CD3 à partir de monocytes afin d'obtenir des cellules négatives pour CD3 ;
   2) congélation des cellules négatives pour CD3 obtenues de l'étape 1) dans un milieu de cryoconservation comprenant du DMSO à 10 % (diméthylsulfoxyde) dans des conditions exemptes de sérum, exemptes de protéines et exemptes de composants animaux pendant 2 mois ou moins, dans lequel la congélation est effectuée par refroidissement graduel allant de -70 °C à -200 °C ;
   3) décongélation des cellules négatives pour CD3 congelées de l'étape 2) ; et
   4) mise en culture des cellules négatives pour CD3 décongelées par traitement des cellules négatives pour CD3 décongelées de l'étape 3) avec IL-15 et IL-21.

【Figure 1】

【Figure 2a】

【Figure 2b】

【Figure 2c】

【Figure 2d】

**Cytotoxicity**

【Figure 2e】

| Frozen cell type | Frozen cell NO. | Cell NO. after thawing | Recovery rate |
|---|---|---|---|
| CD3-cells | $22.5*10^6$ | $17.3*10^6$ | 76.9% |

【Figure 2f】

**Fold induction**

【Figure 2g】

**Viability(tryphan blue)**

【Figure 2h】

a. Degree of differentiation

b. NK receptors (CD94)

【Figure 3a】

V.C (5% HAS)

$1X10^3$ Cells

$1X10^4$ Cells

$5X10^4$ Cells

$1X10^5$ Cells

$5X10^5$ Cells

$1X10^6$ Cells

$5X10^6$ Cells

$1X10^7$ Cells

【Figure 3b】

【Figure 3c】

【Figure 3d】

【Figure 4a】

- Group 1,6: Vehicle control group(+ IL-2)
- Group 2,7 : NK 3X $10^5$ ( + IL-2)
- Group 3,8 : NK 1X $10^6$( + IL-2)
- Group 4,9 : NK 3X $10^6$( + IL-2)
- Group 5,10 : NK 1X $10^7$( + IL-2)

- Group 11 ADR

【Figure 4b】

EP 3 252 152 B1

【Figure 4c】

【Figure 5a】

**Group 1, Group 3 & Group 4**

| 1wk | 2wk | 3wk | 4wk | 5wk |
| D7 | D14 | D21 | D28 | D35 |
| 1st injection | 2nd injection | 3rd injection | 4th injection | End of experiment |

**Group 2, Group 5, Group 6, Group 7 & Group 8**

| 1wk | 2wk | 3wk | 4wk | 5wk |
| D7 D8 | D14 15 | D21 | D28 | D35 |
| 1st 2nd injection | 3rd 4th injection | | | End of experiment |

**Group 9**

| 1wk | 2wk | 3wk | 4wk | 5wk |
| D7 | D14 | D21 | D28 | D35 |
| 1st 2nd 3rd 4th 5th 6th 7th 8th 9th 10th 11th 12th 13th 14th | | | | End of experiment |

【Figure 5b】

once a week for 4 weeks

twice a week for 2 weeks

【Figure 5c】

## once a week for 4 weeks

## twice a week for 2 weeks

【Figure 6a】

【Figure 6b】

【Figure 6c】

【Figure 7a】

【Figure 7b】

【Figure 7c】

【Figure 8a】

G1. Vehicle control group (5% HAS)
G2. NK cells (live, 3x10^5cells/mouse)
G3. NK cells (live, 1x10^6 cells/mouse)
G4. NK cells (live, 3x10^6cells/mouse)
G5. NK cells (live, 1x10^7cels/mouse)
G6. doxorubicin.HCl (in saline)(2mg/kg)

【Figure 8b】

【Figure 8c】

【Figure 8d】

1st V.O

1st 3x10⁵

1st 1x10⁶      H-E Staining, x200      1st 3x10⁶

【Figure 8e】

1st V.O

1st 3x10$^5$

1st 1x10$^6$

CD56, x400

1st 3x10$^6$

【Figure 9a】

**A549**
 G1. Vehicle control group (5% HAS)
 G2. CB NK cells (live, $6\times10^6$ cells/mouse)
 G3. PBL NK cells (live, $6\times10^6$ cells/mouse)
**SNU-709**
 G4. Vehicle control group (5% HAS)
 G5. CB NK cells (live, $6\times10^6$ cels/mouse)
**MIA-paca-2**
 G6. Vehicle control group (5% HAS)
 G7. CB NK cells (live, $6\times10^6$ cels/mouse)

【Figure 9b】

【Figure 9c】

Photograph of A549 tumor on last day (day 25)

Photograph of SNU-709 tumor on last day (day 25)

Photograph of MIA-PaCa-2 tumor on last day (day 25)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20140051263 A

### Non-patent literature cited in the description

- *Ann Rev Immunol.,* 2006, vol. 24, 257-286
- *Trends Immunol.,* 2001, vol. 22, 633-640
- **TILDEN. A.B. et al.** *J. Immunol.,* 1986, vol. 136, 3910-3915
- **BORDIGNON C. et al.** *Hematologia,* 1999, vol. 84, 1110-1149
- **KONJEVIC G. et al.** *Breast Cancer Res. Treat.,* 2001, vol. 66, 255-263
- **RYUKE Y. et al.** *Melanoma Res.,* 2003, vol. 13, 349-356
- **VILLEGAS FR. et al.** *Lung Cancer,* 2002, vol. 35, 23-28
- *Blood Cells Molecules & Disease,* 2004, vol. 33, 261-266
- *Cancer Immunol. Immunother.,* 2007, vol. 56 (11), 1733-1742
- *Breast Cancer Res. Treat.,* 2007, vol. 104 (3), 267-275
- **GALY et al.** *Immunity,* 1995, vol. 3, 459-473
- **MROZEK E et al.** *Blood,* 1996, vol. 87, 2632-2640
- **SIVORI, S. et al.** *Eur J Immunol.,* 2003, vol. 33, 3439-3447
- **B. GRZYWACZ et al.** *Blood,* 2006, vol. 108, 3824-3833
- **SINGER, B et al.** *Proc. Natl. Acad. Sci. USA,* 1995, vol. 92, 377-381
- **SHIBUYA, A. et al.** *Blood,* 1995, vol. 85, 3538-3546
- **DISANTO, J. P et al.** *J. Exp. Med.,* 1990, vol. 171, 1697-1704
- **KOUETSU et al.** *Nature,* 1998, vol. 391, 700-703
- **MROZEKE et al.** *Blood,* 1996, vol. 87, 2632-2640
- *Nature,* 2005, vol. 5, 688-697
- **RAYNA TAKAKI et al.** *J. Immonol,* 2005, vol. 175, 2167-2173
- **ASAO et al.** *J. Immunol,* 2001, vol. 167, 1-5
- **PARRISH-NOVAK et al.** *Nature,* 2000, vol. 408, 57-63
- **M. STRENGELL et al.** *J Immunol,* 2003, vol. 170, 5464-5469
- **J. BRADY et al.** *J Immunol,* 2004, vol. 172, 2048-2058
- **RAYNA TAKAKI et al.** *J Immunol,* 2005, vol. 175, 2167-2173
- **A. MOROZ et al.** *J Immunol,* 2004, vol. 173, 900-909
- **PARRISH-NOVAK et al.** *Nature,* 2000, vol. 408, 57
- **J. BRADY et al.** *J Immunol,* 2004, vol. 172, 2048
- *Remington's Pharmaceutical Science.* Mack Publishing Company